(19) Europäisches Patentamt
European Patent Office
Office européen des brevets

(11) **EP 2 554 680 B1**

(12) **EUROPEAN PATENT SPECIFICATION**

(45) Date of publication and mention
of the grant of the patent:
**12.10.2016 Bulletin 2016/41**

(21) Application number: **11762732.3**

(22) Date of filing: **25.03.2011**

(51) Int Cl.:
*C12Q 1/68* (2006.01)   *C12N 15/09* (2006.01)
*G01N 21/78* (2006.01)   *G01N 33/53* (2006.01)
*G01N 33/533* (2006.01)

(86) International application number:
**PCT/JP2011/057475**

(87) International publication number:
**WO 2011/122501 (06.10.2011 Gazette 2011/40)**

(54) **METHOD FOR DISTINGUISHING TARGET BASE SEQUENCE**

VERFAHREN ZUR UNTERSCHEIDUNG VON ZIELBASISSEQUENZEN

MÉTHODE DE DISTINCTION D'UNE SÉQUENCE DE BASES CIBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.03.2010 JP 2010075297**

(43) Date of publication of application:
**06.02.2013 Bulletin 2013/06**

(73) Proprietors:
• **Toppan Printing Co., Ltd.
Tokyo 110-8560 (JP)**
• **Kyushu University,
National University Corporation
Fukuoka-shi, Fukuoka 812-8581 (JP)**

(72) Inventors:
• **KITANO, Shiro
Tokyo 110-8560 (JP)**
• **YAMANE, Akio
Tokyo 110-8560 (JP)**
• **MARUYAMA, Atsushi
Fukuoka-shi
Fukuoka 812-8581 (JP)**

(74) Representative: **Bailey, Jennifer Ann et al
Marks & Clerk LLP
Alpha Tower
Suffolk Street Queensway
Birmingham B1 1TT (GB)**

(56) References cited:
**EP-A1- 0 664 339      WO-A1-95/02068**

US-A1- 2004 265 823

• KIM W J ET AL: "Comb-type cationic copolymer expedites DNA strand exchange while stabilizing DNA duplex", CHEMISTRY - A EUROPEAN JOURNAL, WILEY - V C H VERLAG GMBH & CO. KGAA, WEINHEIM, DE, vol. 7, no. 1, 1 January 2001 (2001-01-01) , pages 176-180, XP002960295, ISSN: 0947-6539, DOI: 10.1002/1521-3765(20010105)7:1<176::AID-CHEM176>3.0.CO;2-M
• S. W. CHOI ET AL: "Activation of DNA strand exchange by cationic comb-type copolymers: effect of cationic moieties of the copolymers", NUCLEIC ACIDS RESEARCH, vol. 36, no. 1, 13 November 2007 (2007-11-13), pages 342-351, XP055073296, ISSN: 0305-1048, DOI: 10.1093/nar/gkm1035
• KIM W J ET AL: "DNA strand exchange stimulated by spontaneous complex formation with cationic comb-type copolymer", JOURNAL OF THE AMERICAN CHEMICAL SOCIETY, ACS PUBLICATIONS, US, vol. 124, no. 43, 1 January 2002 (2002-01-01), pages 12676-12677, XP002904115, ISSN: 0002-7863, DOI: 10.1021/JA0272080
• MAEDA S. ET AL.: 'Detection of Clarithromycin-Resistant Helicobacter pylori Strains by a Preferential Homoduplex Formation Assay.' JOURNAL OF CLINICAL MICROBIOLOGY vol. 38, no. 1, 2000, pages 210 - 214, XP008166525

EP 2 554 680 B1

- ATSUSHI MARUYAMA: 'Biomaterials and nucleic acid technology' JOURNAL OF JAPANESE SOCIETY FOR BIOMATERIALS vol. 26, no. 6, 2008, pages 433 - 440, XP008166692
- ATSUSHI MARUYAMA: 'Bunshi Chaperone Kogaku ni Motozuku Idenshi Kaiseki' JAPAN SCIENCE AND TECHNOLOGY AGENCY(JST) SENRYAKUTEKI SOZO KENKYU SUISHIN JIGYO(CREST) DAI 4 KAI KOKAI SYMPOSIUM TAILOR-MADE IRYO O MEZASHITA GENOME JOHO KATSUYO KIBAN GIJUTSU YOSHISHU 2008, pages 25 - 28, XP008166877
- WU L. ET AL.: 'Poly(L-lysine)-graft-dextrancopolymer accelerates DNA hybridization by two orders.' SOFT MATTER vol. 4, 2008, pages 744 - 747, XP008166532
- WO L. ET AL.: 'Effect of poly(L-lysine)-g- dextran copolymers on DNA hybridization.' NUCLEIC ACIDS SYMPOSIUM SERIES vol. 51, 2007, pages 73 - 74, XP008166526
- KIM WJ. ET AL.: 'Cationic comb-type copolymers for DNA analysis.' NATURE MATERIALS vol. 2, 2003, pages 815 - 820, XP002904116
- SHIMADA N ET AL.: 'Novel analysis for single nucleotide polymorphism using cationic comb-type copolymers.' NUCLEIC ACIDS SYMPOSIUM SERIES 2007, pages 339 - 340, XP008166527
- S. W. Choi ET AL: "Evaluation of comb-type copolymers with different cationic groups as artificial nucleic acid chaperones", Nucleic Acids Symposium Series, vol. 48, no. 1, 1 November 2004 (2004-11-01), pages 273-274, XP055118602, ISSN: 0261-3166, DOI: 10.1093/nass/48.1.273
- M. HIRANO ET AL: "Analysis of cationic comb-type copolymers/DNA interaction by the single molecular observation and intermolecular force measurement", NUCLEIC ACIDS SYMPOSIUM SERIES, vol. 52, no. 1, 1 September 2008 (2008-09-01), pages 715-716, XP055118605, ISSN: 0261-3166, DOI: 10.1093/nass/nrn361
- TEROUANNE B: "QUANTITATIVE AND QUALITATIVE ANALYSIS OF AMPLIFIED DNA SEQUENCES BY A COMPETITIVE HYBRIDIZATION ASSAY", ANALYTICAL BIOCHEMISTRY, ACADEMIC PRESS INC, NEW YORK, vol. 205, no. 2, 1 September 1992 (1992-09-01), pages 193-199, XP000296793, ISSN: 0003-2697, DOI: 10.1016/0003-2697(92)90423-5

SEQUENCE LISTING

    <110> TOPPAN Printing Co., Ltd.
    <120> Method for identifying target nucleic acid sequence
    <130> PC-14103
    <150> JP2010-075297
    <151> 2010-03-29
    <160> 14
    <170> PatentIn version 3.1
    <210> 1
    <211> 54
    <212> DNA
    <213> Homo sapiens
    <400> 1
    tataaacttg tggtagttgg agctggtggc gtagataaga gtgccttgac gata          54
    <210> 2
    <211> 54
    <212> DNA
    <213> Homo sapiens
    <400> 2
    tatcgtcaag gcactcttat ctacgccacc agctccaact accacaagtt tata          54
    <210> 3
    <211> 54

<212> DNA

<213> Homo sapiens

<400> 3

tataaacttg tggtagttgg agctagtggc gtagataaga gtgccttgac gata 54

<210> 4

<211> 54

<212> DNA

<213> Homo sapiens

<400> 4

tatcgtcaag gcactcttat ctacgccact agctccaact accacaagtt tata      54

<210> 5

<211> 24

<212> DNA

<213> Artificial Sequence

<220>

<223> Description of Artificial Sequence: KF primer

<400> 5

tataaacttg tggtagttgg agct 24

<210> 6

<211> 21

<212> DNA

<213> Artificial Sequence

<220>

<223> Description of Artificial Sequence: KR primer

<400> 6

tatcgtcaag gcactcttgc c 21

<210> 7

<211> 64

<212> DNA

<213> Homo sapiens

<400> 7

```
aagagttggg cgagtacggg ctgcaggcat acactaaagt gaaaactgtg agtgtgggac    60
cttt                                                                 64
```

<210> 8

<211> 64

<212> DNA

<213> Homo sapiens

<400> 8

```
aaaggtccca cactcacagt tttcacttta gtgtatgcct gcagcccgta ctcgcccaac    60
tctt                                                                 64
```

<210> 9

<211> 64

<212> DNA

<213> Homo sapiens

<400> 9

```
aagagttggg cgagtacggg ctgcaggcat acactgaagt gaaaactgtg agtgtgggac    60
cttt                                                                 64
```

<210> 10

<211> 64

<212> DNA

<213> Homo sapiens

<400> 10

```
aaaggtccca cactcacagt tttcacttca gtgtatgcct gcagcccgta ctcgcccaac  60
tctt                                                              64
```

<210> 11
<211> 188
<212> DNA
<213> Homo sapiens
<400> 11

```
tcaaattaca gggtcaactg ctatgatgtg tttggagccc agtcacccct tggtggctac  60
aagatgtcgg ggagtggccg ggagtggccg ggagttgggc gagtacgggc tgcaggcata 120
cactgaagtg aaaactgtga gtgtgggacc tgctgggggc tcagggcctg ttggggcttg 180
agggtctg                                                         188
```

<210> 12
<211> 188
<212> DNA
<213> Homo sapiens
<400> 12

```
tcaaattaca gggtcaactg ctatgatgtg tttggagccc agtcacccct tggtggctac  60
aagatgtcgg ggagtggccg ggagtggccg ggagttgggc gagtacgggc tgcaggcata 120
cactaaagtg aaaactgtga gtgtgggacc tgctgggggc tcagggcctg ttggggcttg 180
agggtctg                                                         188
```

<210> 13
<211> 20
<212> DNA
<213> Artificial Sequence
<220>
<223> Description of Artificial Sequence: primer F
<400> 13
aagagttggg cgagtacggg 20
<210> 14
<211> 24
<212> DNA
<213> Artificial Sequence
<220>
<223> Description of Artificial Sequence: primer R
<400> 14
aaaggtccca cactcacagt tttc 24

**Description**

TECHNICAL FIELD

The present invention relates to a method for identifying whether or not a nucleic acid contained in a sample is a nucleic acid having a target base sequence, and more particularly, to a method that makes it possible to improve nucleic acid identification and shorten reaction time in competitive hybridization.

BACKGROUND ART

Information relating to the human genome is continuing to increase due to the International Hapmap Project for deciphering the human genome and particularly for creating maps of single nucleotide polymorphisms (SNP). Moreover, research is being deployed on a large scale throughout the world towards the realization of "medicine corresponding to personal genetic information" (order-made medicine) that makes it possible to diagnose, treat and prevent illnesses and administer medicine corresponding to personal characteristics by determining the relationship between obtained genome information and individual physical characteristics and gaining an understanding of differences in individual physical characteristics at the genetic level. Genetic differences as referred to here refer to differences in the base sequences of the genomes of individuals, and the primary differences lie in single nucleotide polymorphisms (SNP). More recently, differences in the number of times short base sequences are repeated (number of copies) (copy number variation: CNV) have been determined to extend throughout the entire genome, and this copy number variation has been indicated as being related to disease.

Here, in order to understand differences between individuals at the genetic level, it becomes necessary to investigate the genotype of each individual. For example, in the case of a certain SNP, there have been determined to be three types of genotypes consisting of AA, AG and GG. A indicates adenine, while G indicates guanine, and this SNP is an example in which its locations in the genome are adenine and guanine. Thus, testing for identifying the genotype of this SNP consists of determining which of the three genotypes this SNP corresponds to. Namely, a determination is made as to whether A is 0 or 100, G is 0 or 100, or A and G are 50 and 50.

In this manner, detection of germ line mutations such as SNP is made qualitatively in nearly all cases, the methods are comparatively easy, and various simple methods have come to be used practically.

On the other hand, in cancer cells, mutations occur at the somatic cell level, and those mutations are thought to trigger the onset of cancer and lead to abnormal growth. Thus, mutations of specific genes are observed in certain specific types of cancer cells, and cancer cells can be detected by using those mutations as markers. However, cancer cells are extremely diverse, and it is not necessary easy to specify cancer cells with a single type of mutation.

In addition, in recent drug therapy, drugs have been developed that target a specific molecule in the body (such as protein), and drugs have been discovered that have few adverse side effects and high efficacy. These drugs are referred to as molecular target drugs, and have been actively developed mainly for use in the field of cancer therapy. More recently, it has been determined that, in the case a mutation occurs in a downstream protein of signal transduction of the target molecule, the efficacy of these molecular target drugs may be unable to be demonstrated. In this case, the efficacy of the drug can be predicted by investigating for a mutation in the gene that encodes the mutated protein, thus opening up a new field of order-made medicine that differs from detection of SNP.

The mutations characteristic to cancer cells or mutations that demonstrate resistance to molecular target drugs as mentioned here consist nearly entirely of somatic cell mutations. In contrast to common mutations being observed in all cells in the case of the previously mentioned germ line mutations, in the case of somatic cell mutations, mutations are observed only in those cells that have undergone mutation, and are not observed in cells that have not undergone mutation (typically, normal cells). Thus, mutated cells and normal cells are typically both present in a specimen (sample being tested), and mutated genes and normal genes are both present corresponding to the ratio at which these cells are present.

In other words, in the case the majority of a sample consists of normal cells while containing only a small number of mutated cells, mutant genes only present in a relatively small quantity among the large number of normal genes must be detected, and this constitutes the difference between detecting mutations in somatic cell genes and detecting mutations in the germ line, and is also responsible for the difficulty associated with detecting gene mutations in somatic cells.

Methods for detecting gene mutations in somatic cells can be broadly classified into two types of methods. The first methods involves distinguishing between normal genes and mutant genes at the stage of gene amplification in the detection process, and more specifically, involves specific amplification of mutant genes only.

For example, a method that is considered to have the highest sensitivity is a method known as "mutant-enriched PCR" in which only normal genes are cut using a restriction enzyme, while only uncut mutant genes are amplified (see, for example, Non-Patent Document 1). In this method, by repeating a reaction that amplifies mutant genes, a single molecule of mutant gene can be detected among $10^6$ molecules of normal genes (see, for example, Non-Patent Document 2). Although this method is superior in terms of sensitivity, the procedure is extremely complex and cannot be applied to routine diagnosis.

In addition, a method has been developed for amplifying by distinguishing single nucleotide differences in PCR and other primer extension reactions. This method is referred to as amplification refractory mutation system (ARMS) (see, for example, Non-Patent Document 3) or allele specific PCR (ASPCR) (see, for example, Non-Patent Document 4). Since this method has comparatively high sensitivity, does not require a procedure other than an ordinary PCR amplification reaction, and the entire reaction can be carried out in a closed system, it is extremely simple and is superior in terms of the absence of PCR carryover contamination. However, in the case a normal gene has been amplified even once due to incorrect single base identification, the normal gene ends up being amplified in the same manner as amplification of mutant genes throughout the remaining amplification reaction, thereby resulting in a high risk of false positives. In the case of using this method, it is necessary to precisely control the reaction conditions, namely the reaction temperature, salt concentration and the like, and it is also necessary make the amounts of template exactly the same (see, for example, Non-Patent Document 5). Thus, this method is unsuitable for clinical testing involving testing of an unspecific large number of specimens or diagnoses requiring a high level of accuracy.

Another method for detecting gene mutations in somatic cells consists of simultaneously amplifying mutant genes and normal genes in the detection process, and then subsequently detecting mutant genes and normal genes by distinguishing between the two. Examples of methods used to detect amplified mutant genes and normal genes by distinguishing between the two include methods using electrophoresis and various types of methods using hybridization (see, for example, Non-Patent Document 5). However, in nearly all of these methods, it is difficult to accurately detect a small amount of mutant gene contained among a large amount of normal genes. For example, dideoxy sequencing is considered to be the gold standard for mutant gene detection. Although the dideoxy sequencing method enables detection of a mutant gene with comparatively high sensitivity, in the case both mutant genes and normal genes are present, the detection sensitivity for mutant genes is about 10%, which is inadequate in terms of the level of sensitivity desired in actual testing. In addition, the pyrosequencing method is able to enhance detection sensitivity to about 5%, and has been reported to be superior to the dideoxy sequencing method (see, for example, Non-Patent Document 6).

Moreover, a method has also been developed for determining the ratio of a mutant gene from differences in the melting curves of a mutant gene and a normal gene by amplifying a sequence containing a mutation by PCR and determining the melting curve of double-stranded DNA of the product. In this method as well, a mutant gene contained in normal genes has been reported to be able to be detected up to about 5% (see, for example, Non-Patent Document 7).

A method has also been reported for detecting single nucleotide differences by accurately controlling stringency, for example (see, for example, Non-Patent Document 8). This method utilizes the fact that the melting temperature of an assembly decreases more in the case even one non-complementary base pair is contained between an oligonucleotide probe and a target sequence (mismatch) than in the case the probe is completely complementary with a target sequence (full match). Although this method is also used in DNA arrays and the like, the ability to identify a single base is greatly affected by such factors as the base sequence of the probe, and since this method also requires extremely precise temperature control, it is not necessarily easy to apply to clinical diagnosis.

In addition, PCR-PHFA has been developed as a method for precisely distinguishing single nucleotide differences that uses competition hybridization between two strands having the same base sequence. If the base sequences of a sample targeted for identification of genotype (double-stranded nucleic acid) and a reference double-stranded nucleic acid having a known sequence are completely identical, respective strands are unable to be distinguished and strand recombination (strand displacement) occurs by hybridization. In contrast, if the sample and the reference double-stranded nucleic acid differ by even one base, since the strands having a completely complementary base sequence preferentially forma double strand by competitive hybridization, strand recombination does not occur between the sample and reference double-stranded nucleic acid as a result thereof. The PCR-PHFA method is a mutation detection method that utilizes the above facts. The use of this PCR-PHFA has been reported to enable detection of mutant genes in actual specimens at a high level of sensitivity of about 1% (see, for example, Patent Document 1 and Non-Patent Document 9).

Several variations of the PCR-PHFA method have been proposed. For example, Patent Document 2 discloses a variation of the PCR-PHFA method that uses fluorescence resonance energy transfer. The PCR-PHFA method accurately measures a trace amount of mutant gene with high sensitivity, and although it requires detection of strand recombination between two double-stranded nucleic acids having the same sequence, there are many cases in which the double-stranded nucleic acid of the sample is non-labeled while the reference nucleic acid having a known sequence for inducing strand recombination is labeled. In the method described in Patent Document 2, one strand of the reference nucleic acid is labeled by bonding a fluorescent substance to the vicinity of the 5' end thereof, while the vicinity of the 3' end of the other strand is labeled with a different fluorescent substance. In the case the reference nucleic acid has returned to the original double-stranded state without the occurrence of strand recombination due to competitive hybridization, fluorescence resonance energy transfer is observed between the two different fluorescent substances. In contrast, if strand recombination occurs with the double-stranded nucleic acid of the sample, fluorescence resonance energy transfer is not observed. Thus, the degree of strand recombination can be measured by measuring the degree of this fluorescence resonance energy transfer.

More specifically, in the case of, for example, distinguishing between the case of a certain location of a certain nucleic acid sequence (location of mutation targeted for identification) being adenine and the case of being guanine, a reference double-stranded nucleic acid is prepared that contains that position and in which the base at that position is adenine (and thymine in the complementary strand thereof). Moreover, one strand of this reference double-stranded nucleic acid is labeled with a fluorescent substance X, while the other strand is labeled with a fluorescent substance Y that enables energy transfer with the fluorescent substance X. In other words, since two fluorescent substances are in close proximity in the reference double-stranded nucleic acid, they are in a state that allows transfer of fluorescence resonance energy to occur while in that state.

On the other hand, a nucleic acid originating in a sample is prepared by amplifying so as to have exactly the same length as the reference double-stranded nucleic acid by a nucleic acid amplification reaction. The resulting sample double-stranded nucleic acid and the reference double-stranded nucleic acid are then mixed and then heated to denature the double strands, followed by gradually lowering the temperature to again form double strands. At this time, in the case the mutation sites of the sample-derived double-stranded nucleic acid are all adenine in the same manner as the reference double-stranded nucleic acid, a strand recombination reaction occurs between the sample-derived double-stranded nucleic acid and the reference double-stranded nucleic acid. Theoretically, in the case the ratio of the number of molecules of the sample-derived double-stranded nucleic acid to the number of molecules of the reference double-stranded nucleic acid is 1:1, the probability of recombination is 1/2, the probability of returning to the original double strand is 1/2, and the degree of fluorescence resonance energy transfer is 1/2. In the case mutation sites of the sample-derived double-stranded nucleic acid are all guanine differing from the reference double-stranded nucleic acid, strand recombination does not occur, and thus there is no change in the degree of fluorescence resonance energy transfer. On the basis thereof, a target base desired to be detected (identified) in a sample can be determined to be adenine or guanine. The degree of recombination can be increased by increasing the ratio of the sample-derived double-stranded nucleic acid to the reference double-stranded nucleic acid. For example, in the case the ratio of the sample-derived double-stranded nucleic acid to the reference double-stranded nucleic acid is 20:1, the recombination ratio becomes 20/21, namely the probability of the reference double-stranded nucleic acid returning to its original double strand is 1/21, thereby increasing the magnitude of the change in fluorescence resonance energy transfer and facilitating detection.

In this manner, although the PCR-PHFA method has high detection sensitivity and superior reproducibility, it has the shortcoming of requiring a long period of time. For example, in the methods described in Patent Document 1 and Non-Patent Document 9, in order to accurately carry out competitive hybridization in the PCR-PHFA method, it is necessary to carry out hybridization by applying an extremely gradual temperature gradient (0.1°C/minute) starting at the denaturation temperature of DNA to 70°C or less temperature at which hybridization is completed. This is because, since fluorescent PHFA is a non-enzymatic reaction, strand exchange efficiency between a reference double-stranded nucleic acid and sample double-stranded nucleic acid is governed by thermodynamics, and extreme temperature changes are said to have a detrimental effect on identification accuracy (see, for example, Non-Patent Document 10). In other words, PHFA requires several hours to identify a nucleic acid with adequate accuracy, and this creates a considerable problem in terms of practical application of mutation testing using this method.

On the other hand, a method has been developed that uses a cationic polymer to accelerate the reaction rate of a strand displacement reaction between a double-stranded nucleic acid and single-stranded nucleic acid (see, for example, Patent Document 3). Moreover, methods have been disclosed for detecting single nucleotide differences by utilizing a strand displacement reaction between a single-stranded nucleic acid serving as a sample and a double-stranded nucleic acid serving as a reference (standard) or a strand displacement reaction between a double-stranded nucleic acid serving as a sample and a partial double-stranded nucleic acid serving as a reference under an isothermal environment, by taking advantage of the reaction acceleration effect on the strand displacement reaction afforded by a cationic polymer in this manner (see, for example, Patent Documents 4 and 5). These methods utilizes the fact that, in the case the base sequence of a sample nucleic acid is completely complementary to the base sequence of a reference nucleic acid (full match), the reaction rate of the strand displacement reaction between the two is faster than in the case even one non-complementary base pair is contained (mismatch). Although these methods are superior in terms of their ability to identifying base differences, since they detect a difference of the reaction rates, they have the problem of requiring an excessively long reaction time so as to secure a desired accuracy in detecting single nucleotide differences. In addition, the method described in Patent Document 4 requires that the sample nucleic acid be a single-stranded nucleic acid, while the method of Patent Document 5 requires that a portion for identifying a base be present at a location in the terminal portion of a double-stranded nucleic acid in the double-stranded nucleic acid serving as the sample, and the preparation of such a sample double-stranded nucleic acid is difficult from the viewpoint of practical use.

[Prior Art Documents]

[Patent Documents]

[Patent Document 1] Japanese Patent Publication No. 2982304
[Patent Document 2] Japanese Unexamined Patent Application, First Publication No. 2003-174882
[Patent Document 3] Japanese Unexamined Patent Application, First Publication No. 2001-78769
[Patent Document 4] Japanese Patent Publication No. 4178194
[Patent Document 5] Japanese Unexamined Patent Application, First Publication No. 2008-278779

[Non-Patent Documents]

[Non-Patent Document 1] Chen, et al., Analytical Biochemistry, 1991, Vol. 195, p. 51-56.
[Non-Patent Document 2] Jacobson, et al., Oncogene, 1994, Vol. 9, p. 553-563.
[Non-Patent Document 3] Newton, et al., Nucleic Acids Research, 1989, Vol. 17, p. 2503-2516.
[Non-Patent Document 4] Wu, et al., Proceedings of the National Academy of Sciences of the United States of America, 1989, Vol. 86, p. 2757-2760.
[Non-Patent Document 5] Nollau, etal., Clinical Chemistry, 1997, Vol. 43, p. 1114-1128.
[Non-Patent Document 6] Ogino, et al., The Journal of Molecular Diagnostics, 2005, Vol. 7, p. 413-421.
[Non-Patent Document 7] Krypuy, et al., BMC Cancer, 2006, Vol. 6, p. 295.
[Non-Patent Document 8] Wallace, et al., Proceedings of the National Academy of Sciences of the United States of America, 1983, Vol. 80, p. 278-282.
[Non-Patent Document 9] Tada, etal., Clinica Chimica Acta, 2002, Vol. 324, p. 105.
[Non-Patent Document 10] Oka, et al., Nucleic Acids Research, 1994, Vol. 22, Issue 9, p. 1541-1547.

DISCLOSURE OF THE INVENTION

[Problems to be Solved by the Invention]

With the foregoing in view, an object of the present invention is to provide a method for identifying a base sequence accompanying competitive hybridization such as PCR-PHFA, wherein a nucleic acid contained in a sample can be identified as to whether or not it is a nucleic acid having a target base sequence in an extremely short period of time as compared with conventional methods while maintaining the accuracy of identifying differences in base sequences.

[Means for Solving the Problems]

As a result of conductive extensive studies to solve the aforementioned problems, the inventors of the present invention found that, by adding a cationic comb-type polymer to a reaction solution in a competitive hybridization method such as PCR-PHFA, a base sequence can be identified without impairing identification accuracy even if the temperature is lowered at a rate that exceeds the equilibration rate in competitive hybridization between a sample nucleic acid and reference nucleic acid, thereby leading to completion of the present invention.
Namely, the present invention provides:

(1) a method for identifying a target base sequence, according the appended claim 1. The method comprises:

a thermal denaturation step for subjecting a sample double-stranded nucleic acid and a reference double-stranded nucleic acid containing the same base sequence as a target base sequence to thermal denaturation treatment in a single reaction solution,
a temperature lowering step for carrying out competitive hybridization between the sample double-stranded nucleic acid and the reference double-stranded nucleic acid by lowering the temperature of the reaction solution after the thermal denaturation step,
a measurement step for measuring a double-stranded nucleic acid formed by a nucleic acid strand that composed the reference double-stranded nucleic acid and a nucleic acid strand that composed the sample double-stranded nucleic acid, and

an identification step for identifying identity between the reference double-stranded nucleic acid and the sample double-stranded nucleic acid based on measurement results obtained from the measurement step; wherein,

the temperature lowering step is carried out in the presence of a cationic comb-type polymer

and the temperature lowering rate of the reaction solution in the temperature lowering step is 0.2°C/second to 3°C/second;

(2) the method for identifying a target base sequence described in (1) above, wherein the cationic comb-type polymer has a main chain that is a polymer chain containing a cationic group and a side chain that is a hydrophilic group;

(3) the method for identifying a target base sequence described in (2) above, wherein the main chain of the cationic comb-type polymer is polylysine;

(4) the method for identifying a target base sequence described in (2) or (3) above, wherein the side chain of the cationic comb-type polymer is dextran;

(5) the method for identifying a target base sequence described in any of (2) to (4) above, wherein the main chain of the cationic comb-type polymer contains a guanidyl group;

(6) the method for identifying a target base sequence described in any of (2) to (5) above, wherein the molecular weight of the main chain moiety of the cationic comb-type polymer is 5,000 or more;

(7) the method for identifying a target base sequence described in any of (1) to (6) above, wherein the strand lengths of the sample double-stranded nucleic acid and the reference double-stranded nucleic acid are equal;

(8) the method for identifying a target base sequence described in any of (1) to (7) above, wherein the target base sequence is a base sequence that is homologous with a region containing a mutation site of a specific genotype of a gene mutation;

(9) the method for identifying a target base sequence described in any of (1) to (8) above, wherein the two nucleic acid strands that compose the reference double-stranded nucleic acid are respectively labeled with mutually different types of labeling substances;

(10) the method for identifying a target base sequence described in (9) above, wherein among the two nucleic acid strands that compose the reference double-stranded nucleic acid, energy transfer is possible between a labeling substance used to label one of the nucleic acid strands and a labeling substance used to label the other nucleic acid strand;

(11) the method for identifying a target base sequence described in described in (9) or (10) above, wherein the two nucleic acid strands that compose the reference double-stranded nucleic acid are both labeled with a fluorescent substance;

(12) the method for identifying a target base sequence described in described in (9) or (10) above, wherein among the two nucleic acid strands that compose the reference double-stranded nucleic acid, one of the nucleic acid strands is labeled with a fluorescent substance, while the other nucleic acid strand is labeled with a quenching substance;and

(13) the method for identifying a target base sequence described in any of (9) to (12) above, wherein among the two nucleic acid strands that compose the reference double-stranded nucleic acid, one of the nucleic acid strands is labeled with a labeling substance able to bind to a solid phase carrier.

A kit used in a method for identifying a target base sequence, may comprise:

a reference double-stranded nucleic acid containing the same base sequence as a target base sequence, and a cationic comb-type polymer.

In the kit used in a method for identifying a target base sequence described above, the target base sequence may be a base sequence homologous with a region containing a mutation site of a specific genotype of a gene mutation.

[Effects of the Invention]

According to the method for identifying a target base sequence of the present invention, the amount of time required for competitive hybridization can be dramatically shortened in comparison with conventional methods without impairing identification accuracy.

BRIEF DESCRIPTION OF THE DRAWINGS

FIG. 1 is a drawing schematically showing an example of competitive hybridization between a reference double-stranded nucleic acid and a sample double-stranded nucleic acid having a base sequence differing from that of the reference double-stranded nucleic acid in the identification method of the present invention and a conventional method.

FIG. 2 is a drawing showing real-time measurement of fluorescence intensity of a reaction solution at various temperatures in Example 1.

FIG. 3 is an enlarged representation of third temperature lowering (96°C/minute) in FIG. 2(B).

FIG. 4 depicts graphs showing the results of real-time measurement of fluorescence intensity of a reaction solution at various temperatures in the case of using a wild-type labeled reference double-stranded nucleic acid in Example 2.

FIG. 5 shows the results of rapidly lowering temperature (2.5°/second) in Example 3 by representing as a bar graph of index values.

## BEST MODE FOR CARRYING OUT THE INVENTION

The "identification of a target base sequence" in the present invention and description of the present application refers to identifying whether or not a nucleic acid contained in a sample is a nucleic acid having a target base sequence.

The "sample double-stranded nucleic acid" in the present invention and description of the present application refers to a double-stranded nucleic acid that is prepared from a sample targeted for detection of whether or not it has a target base sequence, and is composed of two mutually complementary nucleic acid strands (single-stranded nucleic acids). In addition, the "reference double-stranded nucleic acid" refers to a double-stranded nucleic acid composed of a nucleic acid strand containing a base sequence identical to a target base sequence and a nucleic acid having a base sequence complementary to that nucleic acid strand.

Furthermore, in the present invention and description of the present application, in addition to the case of the formation of a double strand nucleic acid in a combination of two mutually complementary single-stranded nucleic acids, a double-strand nucleic acid that has dissociated into single-strand nucleic acids is also referred to as a double-stranded nucleic acid. This is because mutually complementary single-stranded nucleic acids may spontaneously form double strands when both are present in solution.

The method for identifying a target base sequence of the present invention (to also be referred to as the "identification method of the present invention") is a method for identifying whether or not a base sequence of a sample double-stranded nucleic acid contains a target base sequence, wherein a sample double-stranded nucleic acid and a reference double-stranded nucleic acid containing a base sequence identical to the target base sequence are mixed and subjected to thermal denaturation, followed by carrying out competitive hybridization in the presence of a cationic comb-type polymer when carrying out competitive hybridization by lowering the temperature. The addition of a cationic comb-type polymer makes it possible to identify a base sequence with high accuracy even if the temperature lowering rate is increased.

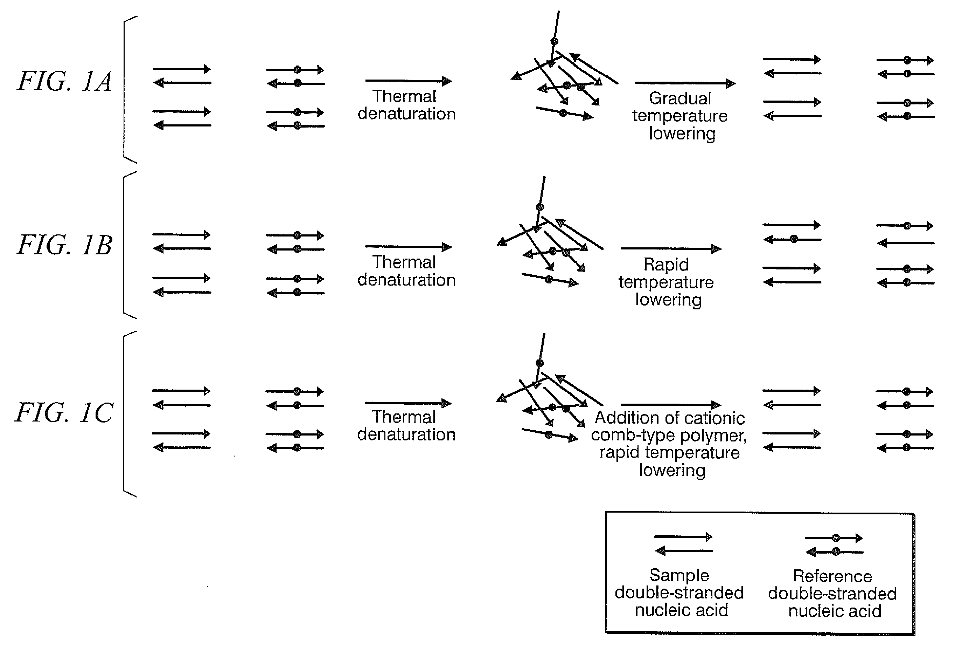

FIG. 1 is a drawing schematically showing an example of competitive hybridization between a reference double-stranded nucleic acid and a sample double-stranded nucleic acid having a base sequence differing from that of the reference double-stranded nucleic acid in the identification method of the present invention and a conventional method. FIG. 1(A) shows the case of carrying out conventional competitive hybridization under gradual temperature lowering conditions, FIG. 1(B) shows the case of carrying out conventional competitive hybridization under rapid temperature lowering conditions, and FIG. 1(C) shows the case of carrying out competitive hybridization of the identification method of the present invention under rapid temperature lowering conditions.

The PCR-PHFA method is an example of a competitive hybridization method. In the PCR-PHFA method, although hybridization reaction is typically carried out while lowering the temperature starting from the thermal denaturation temperature of a double-stranded nucleic acid, it is important that the temperature be lowered gradually (0.1°C/minute). If the temperature lowering rate during the competitive hybridization reaction is gradual, double strand formation between completely complementary nucleic acid strands (homoduplexing) occurs preferentially to double strand formation between nucleic acid strands having different base sequences (heteroduplexing) (FIG. 1(A)). On the other hand, in the case the temperature lowering rate is increased, since the temperature changes prior to the hybridization reaction reaching equilibrium at each temperature, base sequence identification accuracy is inadequate, and not only homoduplex formation, but also heteroduplex formation end up occurring (FIG. 1(B)). In particular, in the case the base sequences of a reference double-stranded nucleic acid and a sample double-stranded nucleic acid only differ by one base, heteroduplexing occurs easily resulting in erroneous identification of mutations. Thus, conventional PCR-PHFA methods had the shortcoming of being unable to easily shorten reaction time.

In contrast, in the identification method of the present invention, since a cationic comb-type polymer is added to the reaction solution, homoduplexing occurs preferentially even in cases in which the temperature of the reaction solution has been lowered rapidly, thereby making it possible to detect whether or not a sample double-stranded nucleic acid has a base sequence identical to a target base sequence with high accuracy. Although the reason for obtaining such an effect as a result of carrying out a competitive hybridization reaction in the presence of cationic comb-type polymer is unclear, it is surmised that equilibrium can be reached quickly as a result of the reaction rate of the hybridization reaction being greatly increased by the presence of the cationic comb-type polymer.

In the identification method of the present invention, in the case a cationic comb-type polymer is not present, a nucleic acid can be identified even in the case different base sequences cannot be distinguished due to a rapid change in temperature. In actuality, in the case a cationic comb-type polymer was added to a reaction solution in Example 1 to be subsequently described, even in the case that a competitive hybridization is carried out on condition that the temperature lowering rate is 100 times faster than that of the usual case, identifiability was maintained between base sequences differing by only one base. On the other hand, in the case a cationic comb-type polymer was not added and the base sequences of a reference double-stranded nucleic acid and a sample double-stranded nucleic acid differed by only one base, erroneous hybridization occurred, or in other words, a large number of heteroduplexes were formed, and it was not possible to distinguish between the case of the base sequences of the reference double-stranded nucleic acid and sample double-stranded nucleic acid being the same (match) or the case of their being different (mismatch). This result is only natural from the viewpoint of thermodynamic equilibrium in the case of not adding a cationic comb-type polymer.

It was first found by the inventors of the present invention that, in the case nucleic acid strands having similar base sequences are present in a reaction solution, nucleic acid strands having completely complementary base sequences preferentially form complementary strands in the presence of a cationic comb-type polymer even if the temperature changes rapidly, which was in considerable defiance of the conventional way of thinking of nucleic acid chemistry and thermodynamics. As a result of this finding, highly precise temperature control conventionally required by competitive hybridization reactions such as PCR-PHFA was no longer required, and measurement time was able to be shortened to less than 1 minute.

Although there are no particular limitations on the cationic comb-type polymer used in the present invention provided it has a comb-like side chain introduced into the main chain and contains a cationic group, it is preferably a polymer in which a comb-like side chain having a hydrophilic group is introduced into polymer having a polymer chain containing a cationic group (polycation) for the main chain thereof, and is more preferably a copolymer in which a comb-like polymer containing a hydrophilic group is introduced into a main chain in the form of a polycation (to be referred to as a cationic comb-type copolymer).

Examples of the polycation serving as the main chain of the cationic comb-type copolymer include polymers obtained by polymerizing one type or a plurality of types of cationic monomers such as amino acids such as lysine, arginine, histidine or ornithine, sugars such as glucosamine, allylamines or ethyleneimines, and derivatives thereof. In the present invention, cationic comb-type copolymers having for the main chain thereof polylysine, polyarginine, copolymers of lysine and arginine or derivatives thereof are used preferably.

Examples of derivatives of polymers of cationic monomers include those in which a functional group has been added to all or a portion of a monomer that composes the polymer. There are no particular limitations on such functional groups provided they do not impair the cationicity of the polymer, and an example of such a functional group is a guanidyl group.

Examples of the polymer containing a hydrophilic group serving as a side chain of the cationic comb-type copolymer include polysaccharides such as dextran, amylose or cellulose, polyethers such as polyethylene glycol, and derivatives thereof. In the present invention, cationic comb-type copolymers having for a side chain thereof dextran, polyethylene glycol or a derivative thereof are used preferably.

There are no particular limitations on the derivatives of the polymer containing a hydrophilic group used as a side chain provides they do not impair hydrophilicity, and examples include carboxymethyl derivatives, amino acid derivatives and aldehyde derivatives.

The cationic comb-type polymer used in the present invention is preferably a cationic comb-type copolymer in which the main chain is polylysine, polyarginine or a derivative thereof, and the side chain is dextran, more preferably a cationic comb-type copolymer in which the main chain is polylysine, polyarginine or a guanidine derivative thereof (derivative containing a guanidyl group) and the side chain is dextran, and even more preferably a cationic comb-type copolymer obtained by graft polymerizing dextran to polylysine or guanidinated polylysine.

The size of the cationic comb-type polymer used in the present invention is suitably selected corresponding to the strand lengths of the sample double-stranded nucleic acid and reference double-stranded nucleic acid used in competitive hybridization. In the present invention, the molecular weight of the main chain moiety is preferably 2,000 or more and more preferably 5,000 or more. For example, in the case of using a cationic comb-type copolymer obtained by graft polymerizing dextran to polylysine or guanidinated polylysine, the molecular weight of the main chain moiety is 2, 000 to 20, 000 and preferably 5,000 to 15,000. Furthermore, a cationic comb-type polymer having a main chain moiety of a desired size can be obtained by adjusting the degree of polymerization of the polycation of the main chain.

Furthermore, as is described in Patent Documents 3 to 5, cationic comb-type polymers are known to have the effect of promoting strand exchange in a strand displacement reaction. However, there is no indication whatsoever in Patent Documents 3 to 5 regarding the action or effect on the formation of a double strand by single-stranded nucleic acids or competitive hybridization. The finding of being able to greatly shorten the time required by a competitive hybridization reaction by increasing the temperature lowering rate of a reaction solution without impairing base sequence identification accuracy as a result of carrying out the reaction in the presence of a cationic comb-type polymer is a finding that was first obtained by the inventors of the present invention.

Here, a strand exchange reaction refers to a reaction in which a single-stranded nucleic acid having a base sequence similar or completely identical to one nucleic acid strand of a double-stranded nucleic acid is replaced with the other nucleic acid strand of a double-stranded nucleic acid that actually forms a double strand. In contrast, a hybridization reaction refers to a reaction in which two single-stranded nucleic acids having complementary base sequences form a double-stranded nucleic acid. Moreover, a competitive hybridization reaction refers to a reaction in which two or more single-stranded nucleic acids competitively hybridize with one single-stranded nucleic acid to form a double-stranded nucleic acid. In the case of carrying out competitive hybridization reaction under optimum reaction conditions, since the single-stranded nucleic acid that forms a more stable double-stranded nucleic acid is preferentially hybridized among the competitive single-stranded nucleic acids, the most stable double-stranded nucleic acid is formed as a result thereof.

The sample double-stranded nucleic acid used in the present invention can be prepared by using, for example, a nucleic acid amplification reaction. There are no particular limitations on the nucleic acid amplification reaction, and can be suitably selected and used from among known nucleic acid amplification reactions such as PCR, ligase chain reaction (LCR), self-sustained sequence replication (3SR) or strand displacement amplification (SDA) (Manak, DNA Probes, 2nd Edition, p. 255-291, Stockton Press (1993)). PCR is used particularly preferably in the present invention since the amplified nucleic acid fragment can be obtained in the form of a double-stranded nucleic acid. Furthermore, in the case the nucleic acid amplification product is not a double-stranded nucleic acid, a step is required for preparing in the form of a double-stranded nucleic acid by using a primer extension reaction and the like.

Although the reference double-stranded nucleic acid used in the present invention can be prepared by using a nucleic acid amplification reaction in the same manner as the sample double-stranded nucleic acid, it is preferably prepared by a known chemical synthesis method. This is because the use of a chemical synthesis method results in a higher degree of freedom with respect to the introduced location of a labeling substance and the like. Examples of chemical synthesis methods include the triester method and phosphite method. For example, double-stranded DNA can be prepared by preparing a large amount of single-stranded DNA using an ordinary automated synthesizer (such as Applied Biosystems Model 392), which uses a liquid phase method or solid phase synthesis method using an insoluble carrier, followed by annealing.

In the identification method of the present invention, although the strand lengths of the sample double-stranded nucleic acid and reference double-stranded nucleic acid may be different, both chain lengths are preferably equal. This is because single nucleotide differences can be identified with higher accuracy since the effects of hybridization efficiency attributable to differences in chain lengths can be suppressed.

More specifically, the identification method of the present invention comprises a thermal denaturation step for subjecting a sample double-stranded nucleic acid and a reference double-stranded nucleic acid containing the same base sequence as a target base sequence to thermal denaturation treatment in a single reaction solution, a temperature lowering step for carrying out competitive hybridization between the sample double-stranded nucleic acid and the reference double-stranded nucleic acid by lowering the temperature of the reaction solution after the thermal denaturation step, a measurement step for measuring a double-stranded nucleic acid formed by a nucleic acid strand that composed the reference double-stranded nucleic acid and a nucleic acid strand that composed the sample double-stranded nucleic acid, and an identification step for identifying identity between the reference double-stranded nucleic acid and the sample double-stranded nucleic acid based on measurement results obtained from the measurement step; wherein, the temperature lowering step is carried out in the presence of a cationic comb-type polymer.

The thermal denaturation step is a step for subjecting a sample double-stranded nucleic acid and a reference double-stranded nucleic acid to thermal denaturation treatment in a single reaction solution. More specifically, after respectively adding a sample double-stranded nucleic acid and a reference double-stranded nucleic acid to a reaction solution having a suitable composition, thermal denaturation treatment is carried out by heating the reaction solution. For example, by heating the reaction solution for a fixed period of time at a temperature of 90°C to 100°C and preferably 95°C to 100°C, the sample double-stranded nucleic acid and reference double-stranded nucleic acid contained in the reaction solution can be denatured. Furthermore, the sample double-stranded nucleic acid and the reference double-stranded nucleic acid may also be added reaction solutions in the form of solutions in which they are respectively contained.

Although the cationic comb-type polymer may be added to the reaction solution prior to the start of the temperature lowering step after thermal denaturation treatment, it is preferably added to the reaction solution before thermal denaturation treatment. The amount of cationic comb-type polymer added to the reaction solution can be determined based on the charge ratio of cationic comb-type polymer to nucleic acid ((charge of cationic comb-type copolymer)/(charge of nucleic acid)) in the reaction solution, and a suitable value is selected for the charge ratio that is within the range of 0.01 to 10,000. Furthermore, the charge of nucleic acid is the sum of the charges of all nucleic acids present in the reaction solution.

In addition, a buffering agent for adjusting the pH of the reaction solution, a monovalent or divalent cation required to form a double strand, or an organic solvent and the like that affects a stability of double-stranded nucleic acids can also be added to the reaction solution. Examples of buffering agents include Tris-hydrochloride. Examples of cations include sodium ions and magnesium ions. Examples of organic solvents include dimethylsulfoxide (DMSO) and dimethylformamide (DMF).

Next, in the temperature lowering step, competitive hybridization is carried out on the sample double-stranded nucleic acid and the reference double-stranded nucleic acid in the presence of a cationic comb-type polymer by lowering the temperature of the reaction solution following the thermal denaturation step. In the temperature lowering step, the temperature is lowered from the denaturation temperature to a temperature equal to or lower than the Tm value of the reference double-stranded nucleic acid, and the temperature of the reaction solution at completion of the reaction can be suitably set corresponding to the strand length and base sequence of the reference double-stranded nucleic acid and sample double-stranded nucleic acid.

In the identification method of the present invention, since the competitive hybridization reaction is carried out in the presence of a cationic comb-type polymer, the reaction can be carried out by lowering the temperature from the denaturation temperature to a temperature equal to or lower than the Tm value of the reference double-stranded nucleic acid at a rapid rate, such as at a rate of 0.2°C/second or more. Furthermore, the competitive hybridization reaction can also be carried out at a more gradual temperature lowering rate than 0.2°C/second. This rapid temperature change can be carried out using a dedicated device enabling temperature control or a real-time PCR device and the like.

In the present invention, the temperature lowering rate of the reaction solution is preferably 0.2°C/second to 3°C/second, more preferably 0.5°C/second to 3°C/second and even more preferably 0.5°C/second to 2°C/second. The amount of time required for the temperature lowering step can be reduced considerably by rapidly lowering the temperature in this manner. Consequently, according to the identification method of the present invention, competitive hybridization can be carried out in about 30 seconds and at most about 60 seconds.

Next, in the measurement step, a double-stranded nucleic acid formed by a nucleic acid strand that composed the reference double-stranded nucleic acid and a nucleic acid strand that composed the sample double-stranded nucleic acid is measured, and in the identification step, identity between the reference double-stranded nucleic acid and the sample double-stranded nucleic acid is identified based on the resulting measurement results. In the subsequent descriptions, the "double-stranded nucleic acid formed by a nucleic acid strand that composed the reference double-stranded nucleic acid and a nucleic acid strand that composed the sample double-stranded nucleic acid" is also referred to as a "double-stranded nucleic acid formed by strand recombination".

In the case an adequate amount of double-stranded nucleic acid formed by strand recombination is present in the reaction solution, the sample double-stranded nucleic acid is judged to be identical to the reference double-stranded nucleic acid and have a target nucleic acid sequence. On the other hand, in the case a double-stranded nucleic acid formed by strand recombination is not present in an adequate amount, the sample double-stranded nucleic acid can be judged to not be identical to the reference double-stranded nucleic acid. Furthermore, in the present invention, the "measuring a double-stranded nucleic acid formed by strand recombination" includes detecting the double-stranded nucleic acid per se formed by strand recombination as well as measuring the ratio of the double-stranded nucleic acid formed by strand recombination to the reference double-stranded nucleic acid.

Furthermore, measurement of the double-stranded nucleic acid formed by strand recombination may be carried out at completion of the temperature lowering step, namely following competitive hybridization (endpoint), or at the start and completion of the temperature lowering step, after which whether or not the sample double-stranded nucleic acid is identical to the reference double-stranded nucleic acid may be identified by comparing both measured values. In addition, the measurement of the measurement step may also be carried out over time (on a real-time basis) during the temperature lowering step.

By labeling either the reference double-stranded nucleic acid or sample double-stranded nucleic acid with a labeling substance in advance, the double-stranded nucleic acid formed by strand recombination can be measured by using the labeling substance as a marker. In general, it is preferable to label the standard double-stranded nucleic acid since this can be prepared in advance. In particular, the two nucleic acid strands that compose the reference double-stranded nucleic acid are preferably each labeled with mutually different labeling substances. In the case of having labeled in advance in this manner, the double-stranded nucleic acid formed by strand recombination is labeled with only one type of labeling substance, and can be detected by distinguishing from the reference double-stranded nucleic acid labeled with two types of labeling substances.

Although either non-radioactive or radioactive substances may be used as labeling substances, non-radioactive substances are used preferably. Examples of non-radioactive labeling substances include substances that can be labeled directly such as fluorescent substances (for example, fluorescein derivatives such as fluorescein isothiocyanate), rhodamine and derivatives thereof (such as tetramethylrhodamine isothiocyanate), and chemiluminescent substances (such as acridine). In addition, labeling substances can also be detected indirectly by using a substance that specifically binds to the labeling substance. Examples of such labeling substances include biotin, ligands and specific nucleic acids or protein haptens. In the case of using biotin as a substance that specifically binds to a labeling substance, avidin or steptoavidin can be used that specifically binds thereto, in the case of using a hapten, an antibody that specifically binds thereto can be used, a receptor can be used in the case of a ligand, and in the case of a specific nucleic acid or protein, nucleic acid-binding protein or protein having affinity for a specific protein can be used. A compound having a 2,4-dinitrophenyl group or digoxigenin can be used for the aforementioned hapten, and biotin or a fluorescent substance can also be used as a hapten. These labeling substances can be introduced by known means either alone or in a combination of multiple types thereof if necessary (see Japanese Unexamined Patent Application, First Publication No. S59-93099, Japanese Unexamined Patent Application, First Publication No. S59-148798 and Japanese Unexamined Patent Application, First Publication No. S59-204200).

In addition, in the case either of two types of labeling substances is able to bind to a solid phase carrier, the degree to which strand recombination occurs between the reference double-stranded nucleic acid and sample double-stranded nucleic acid can be measured by carrying out a commonly used solid-liquid separation procedure. For example, one of the strands of the reference double-stranded nucleic acid is labeled with labeling substance A, the other strand is labeled with labeling substance B capable of binding to a solid phase carrier, and the reaction solution following competitive hybridization is contacted with the solid phase carrier to which the labeling substance B is able to bind. Subsequently, the labeling substance A is detected in the double-stranded nucleic acid bound to the solid phase carrier. In the case strand recombination has occurred, the ratio of double-stranded nucleic acid labeled with labeling substance A decreases in the double-stranded nucleic acid bound to the solid phase carrier. Furthermore, in the case one of the strands of a labeled double-stranded nucleic acid is displaced by a strand that is not labeled, the newly formed double-stranded nucleic acid is able to bind to the solid phase carrier, it cannot be detected due to the absence of a label for detection.

In the present invention in particular, a double-stranded nucleic acid formed by strand recombination is preferably measured by using two types of labeling substances capable of mutual energy transfer (such as a donor labeling substance that generates fluorescence when excited, and an acceptor labeling substance that absorbs the fluorescence), and then using the degree of the change in energy resulting from energy transfer between these labeling substances as a marker.

Energy transfer between labeling substances refers to a transfer of energy from a donor labeling substance to an acceptor labeling substance in the case at least two types of labeling substances consisting of the donor labeling substance, which generates energy, and the acceptor labeling substance, which absorbs energy generated from the donor labeling substance, are in mutually close proximity. For example, in the case two types of labeling substances are fluorescent substances, fluorescence generated by excitation of the donor labeling substance is absorbed by the acceptor labeling substance and fluorescence emitted by the acceptor labeling substance is then measured, or quenching of the donor labeling substance, which occurs as a result of the acceptor labeling substance absorbing fluorescence generated by exciting the donor labeling substance, is measured (PCR Methods and Applications 4, 357-362 (1995), Nature Biotechnology 16, 49-53 (1998)). Furthermore, there are cases in which energy transfer occurs even if there is no overlapping of the fluorescence wavelength of the donor labeling substance and the absorption wavelength of the acceptor labeling substance, and such energy transfer is also included in the present invention. In addition, the acceptor labeling substance may also be a quenching substance. Examples of such quenching substances include DABCYL and black hole quenchers.

Although there are no particular limitations on the two types of labeling substances that allow mutual energy transfer provided they allow energy transfer when the labeling substances are in mutually close proximity, fluorescent substances and delayed fluorescent substances are preferable, and depending on the case, chemiluminescent substances and bioluminescent substances can also be used. Examples of such combinations of labeling substances include combinations of fluorescein or derivatives thereof (such as fluorescein isothiocyanate) and rhodamine or derivatives thereof (such as tetramethylrhodamine isothiocyanate, tetramethylrhodamine-5-(and-6)-hexanoic acid), and combinations of fluorescein and DABCYL, and arbitrary combinations can be selected there from (Nonisotopic DNA Probe Techniques, Academic Press (1992)).

In addition, combinations of molecules that release thermal energy when in close proximity may also be used. Examples of combinations of such labeling substances include combinations of a substance selected from the group consisting of Alexa Fluor™ 488 (Invitrogen), Atto 488 (Atto-Tec GmbH), Alexa Fluor™ 594 (Invitrogen) and ROX (Carboxy-X-Rhodamine) with BHQ™ (black hole quencher)-1 or BHQ™-2.

Furthermore, guanine may also be used since it has the ability to quench when FAM is in close proximity (Nucleic Acids Research, 2002, Vol. 30, No. 9, p. 2089-2195). For example, in the case the 3' end part of one strand of a reference double-stranded nucleic acid is labeled with FAM and the base on the 5' end of the other stand is guanine, the other strand is not required to be labeled with a labeling substance.

A method commonly employed for introducing a label into a nucleic acid can be used for the method used to introduce the labeling substance into the reference double-stranded nucleic acid or sample double-stranded nucleic acid. Examples of such methods include a method in which a labeling substance is chemically introduced into a nucleic acid directly (Biotechniques, 24, 484-489 (1998)), a method in which a mononucleotide bound to a labeling substance is introduced by a DNA polymerase reaction or RNA polymerase reaction (Science, 238, 336-3341 (1987)), and a method in which a labeling substance is introduced by carrying out a PCR reaction using a primer into which the labeling substance has been introduced (PCR Methods and Applications, 2, 34-40 (1992)).

The location where the labeling substance is introduced into the reference double-stranded nucleic acid or sample double-stranded nucleic acid is required to be a location where energy transfer attributable to the strand displacement reaction occurs or is quenched, namely the 3' end part and/or 5' end part of a nucleic acid strand. More specifically, in the present invention, although the 5' end part and 3' end part respectively indicate a range within 30 bases from the 5' end and 3' end of a nucleic acid strand, since energy transfer occurs more easily the closer both labeling substances are, the 5' end part and the 3' end part preferably indicate a range within 10 cases from each end and most preferably indicate the 5' end and 3' end. Here, since there is the possibility of being unable to detect displacement on the order of a single base if a large number of labeling substances are introduced in the base moiety that hybridizes with a complementary strand, the labeling substances are preferably only introduced into each end part of the nucleic acid strand. For example, by introducing one of two types of labeling substances into the 5' end part (3' end part) of one nucleic acid strand and introducing the other type of labeling substance into the 3' end part (5' end part) of the other nucleic acid strand complementary thereto, both nucleic acid strands undergo energy transfer or quenching due to a strand displacement reaction without having an effect on the hybridization reaction.

The identification method of the present invention can be preferably used to identify a gene mutation. More specifically, a base sequence of a region containing a mutation site of a gene mutation is used as a target base sequence. Furthermore, in the present invention, a gene mutation refers to a difference in the base sequence of a gene that is present among individuals of the same biological species, while a mutation site refers to the location that differs in the base sequence. More specifically, differences in base sequences occur as a result of substituting, deleting or inserting one or a plurality of bases in a base sequence. Examples of such gene mutations include SNP and CNV polymorphisms. In addition, in the present invention, gene mutations include congenital mutations in the manner of genetic polymorphisms such as SNP as well as acquired mutations in the manner of somatic cell mutations that constitute differences in the base sequence of a gene that is present among cells of the same individual.

In the identification method of the present invention, gene mutations serving as targets refer to mutations in cancer-related genes, genes related to genetic diseases, genes related to drug metabolism and efficacy, viral genes and bacterial genes. Examples of cancer-related genes include KRAS gene, BRAF gene, PTEN gene, PIK3CA gene, ALK fusion gene, EGFR gene, NRAS gene, p53 gene, BRCA1 gene, BRCA2 gene and APC gene. Examples of genes related to genetic diseases include genes reported to be involved in various types of congenital metabolic disorders. Examples of gene related to drug metabolism include genes of enzymes such as cytochrome P450 or transporters involved in drug metabolism. Examples of viral genes and bacterial genes include hepatitis C virus and hepatitis B virus genes. Moreover, genes such as the human leukocyte antigen gene HLA, which are not necessarily directly involved in the cause of a disease, are also important with respect to such factors as transplant compatibility and drug side effects. Moreover, gene mutations encoded by mitochondria have also been suggested to be related to disease, and mutations of these genes can also be targets.

The identification method of the present invention is also useful in clinical laboratory testing and the like in consideration of its high level of identification accuracy and speed. When considering the practicality of genetic testing in the clinical setting, shortening measurement time is extremely important. According to the identification method of the present invention, germ cell mutations such as SNP as well as somatic cell mutations can be identified with high accuracy and in a short period of time.

For example, KRAS is a protein involved in signal transduction that is a proto-oncogene. Mutations have been reported to occur in KRAS gene in numerous cancer cells. Mutations accompanying amino acid substitution at codons 12 and 13 of KRAS gene have been observed particularly prominently, and 13 types of mutation patterns are known to exist. Recently, anticancer drugs in the form of EGFR antibody drugs (such as cetuximab or panitumumab) have been increasingly determined to be unable to demonstrate efficacy in patients having a mutation in KRAS gene. In addition to being associated with adverse side effects, such anti-cancer drug therapy is also extremely expensive. Thus, testing for KRAS gene mutations prior to therapy and performing therapy by screening for only those patients in which therapy is effective has been proposed as a part of order-made medicine.

In addition, the EGFR antibody drug, cetuximab, is used for the treatment of colorectal cancer. The number of persons afflicted with colorectal cancer is currently just under 100,000 persons annually, and the number of resulting deaths reached 40,800 as of 2005. According to estimates by EGFR antibody drug manufacturers, 40, 000 colorectal cancer patients will be targeted for EGFR antibody drug therapy in four years as the trend towards a more Westernized diet continues to increase. If this estimate is correct, the size of the market for KRAS gene testing in Japan alone is predicted to exceed 400 million yen in four years.

However, it is difficult for conventional identification methods to identify somatic cell mutations rapidly with adequate accuracy, and have the shortcoming of being associated with a large number of false positives. Since the identification method of the present invention is able to also rapidly identify somatic cell mutations with extremely high accuracy, it can be expected to contribute to not only improvement of the accuracy of clinical laboratory testing, but also a reduction in health care costs.

The identification method of the present invention can be applied to PCR-PHFA in the manner described below. First, a primer is designed that amplifies a mutated region (target base sequence) desired to be detected, and an amplification product that contains the mutated region is obtained by using a sample nucleic acid as a template. On the other hand, a labeled reference double-stranded nucleic acid is also prepared that contains the same region as the amplification product containing the mutated region and is formed from nucleic acid strands respectively labeled with two types of labeling substances capable of energy transfer.

For example, the 5' end of one nucleic acid strand is labeled with a donor labeling substance such as fluorescein, while the 3' end of the other nucleic acid strand is labeled with an acceptor labeling substance such as DABCYL. These two labeled nucleic acid strands are then mixed to obtain a labeled reference double-stranded nucleic acid. A labeled nucleic acid strand can be prepared using chemical synthesis, including introduction of the label.

In order to actually detect a gene mutation in a sample, a reaction solution is prepared by adding a reaction solution amplified by PCR, labeled reference double-stranded nucleic acid, and cationic comb-type polymer. This reaction solution is then denatured by subjecting to heat treatment at about 90°C, after which competitive hybridization is carried out by lowering the temperature of the reaction solution to about the Tm value of the labeled reference double-stranded nucleic acid, such as about 70°C, at a temperature lowering rate of 0.2°C/second or more, and for example, about 2°C/second, followed finally by measuring fluorescence. The temperature at which fluorescence is measured may be 70°C or in the vicinity of room temperature. In addition, changes in fluorescence during temperature lowering may also be measured successively using a real-time PCR device and the like. Double-stranded nucleic acid formed by strand recombination is then measured based on the resulting measured values to assess the degree of strand recombination and judge whether or not the base sequence of nucleic acid in the sample is the same as that of the labeled reference double-stranded nucleic acid. Measured values can also be corrected by using fluorescence values of the reaction solution prior to thermal denaturation treatment or fluorescence values during thermal denaturation in order to reduce measurement error and the like when measuring fluorescence.

A kit for identifying a target base sequence may comprise a reference double-stranded nucleic acid containing the same base sequence as a target base sequence, and a cationic comb-type polymer. In addition, the kit for identifying a target base sequence may also combine a reagent added to the competitive hybridization reaction solution, such as a buffering agent, cation or organic solvent, and a reagent for detecting the label of a labeling substance. In this manner, by incorporating reagents required by the identification method of the present invention into a kit, identification of a target base sequence can be carried out more easily and in a short period of time.

The kit for identifying a target base sequence is preferably used for detecting a gene mutation by using a base sequence homologous with a region containing a mutation site of a specific genotype of a gene mutation for the target base sequence.

[Examples]

Although the following provides a specific explanation of the present invention by indicating examples thereof, the present invention is not limited to the following examples.

[Example 1]

Using a gene mutation at codon 12 of the cancer gene KRAS for the mutation site targeted for identification, and using the base sequence of a partial region containing the mutation site for the target base sequence, the identification method of the present invention was used to identify whether or not the genotype of a sample double-stranded nucleic acid is identical to the genotype of the reference double-stranded nucleic acid. Furthermore, the reference double-stranded nucleic acid and sample double-stranded nucleic acid used were prepared in accordance with ordinary chemical synthesis methods.

First, reference double-stranded nucleic acids of the codon 12 wild type (Wild) and a mutant type (G12S) were respectively prepared. The mutant type (G12S) is a mutant type in which glycine has been altered to serine due to a point mutation of codon 12. Both ends of one of the nucleic acid strands of each reference double-stranded nucleic acid were labeled with FAM (Glen Research Corp.), while both ends of the other nucleic acid strand were labeled with DABCYL (Glen Research Corp.). Reference double-stranded nucleic acids of each genotype were prepared by hybridizing the products of chemically synthesizing one strand each of the two nucleic acid strands that compose the reference double-stranded nucleic acids. The sequences of the chemically synthesized nucleic acid strands are shown in Table 1 for each genotype. In Table 1, codons 12 and 13 are underlined, and the mutation sites are represented with lower case letters. In addition, "6-FAM" indicates the FAM label, "DAB" indicates the DABCYL label, and the numerals in the right-hand column indicate the corresponding sequence numbers listed in the sequence listing.

In addition, nucleic acid strands having the same base sequence as the base sequences shown in Table 1 but not labeled were also synthesized, and mixtures of mutually complementary nucleic acid strands were used for the sample double-stranded nucleic acid.

[Table 1]

| | | Reference Double-Stranded Nucleic Acids | |
|---|---|---|---|
| Wild | 5'(6-FAM)-_TATAAACTTGTGGTAGTTGGAGCT_GGTGGC_GTAGAT AAGAGTGCCTTGACGATA-(6-FAM)3' | | 1 |
| | 5'(6-DAB)-_TATCGTCAAGGCACTCTTATCTAC_GCCACC_AGCTCC AACTACCACAAGTTTATA-(DAB)3' | | 2 |
| G12S | 5'(6-FAM)-_TATAAACTTGTGGTAGTTGGAGCT_aGTGGC_GTAGAT AAGAGTGCCTTGACGATA-(6-FAM)3' | | 3 |
| | 5'(6-DAB)-_TATCGTCAAGGCACTCTTATCTAC_GCCACt_AGCTCC AACTACCACAAGTTTATA-(DAB)3' | | 4 |

500 nM wild-FAM (single-stranded nucleic acid labeled with FAM of the wild-type reference double-stranded nucleic acid) (1 μL), 500 nM wild-DAB (single-stranded nucleic acid labeled with DABCYL of the wild-type reference double-stranded nucleic acid) (1μL), 2 M NaCl (1 μL), ROX (0.6 μL), 0.5 M EDTA (2 μL), cationic comb-type polymer (1 μL), 1× PCR buffer (12.4 μL) and 5 μM sample double-stranded nucleic acid (2 μL) were mixed for use as a fluorescent PHFA reaction solution. A mixture containing pure water (1 μL) instead of cationic comb-type polymer was used as a control. An ROX solution manufactured by Invitrogen was used.

Furthermore, a polymer comprised of polylysine for the main chain and dextran for the side chain was used for the cationic comb-type polymer, and three types of cationic polymers (CP1 to CP3) were prepared. CP1 was obtained by adding 88% by weight of dextran to a polylysine main chain having a molecular weight of 5,000, CP2 was obtained by adding 88% by weight of dextran to a polylysine main chain having a molecular weight of 15,000, and CP3 was obtained by adding 88% by weight of dextran to a guanidinated polylysine main chain having a molecular weight of 15,000. The total amount of anion was calculated from the phosphate residue concentration of nucleic acids present in the entire reaction solution, and cationic polymer was added to the reaction solution so that the amount of cationic polymer was roughly eight times the amount of anion.

The prepared fluorescent PHFA solution was placed in a real-time PCR device (ABI-7900) and held for 15 minutes at 35°C, followed by continuously measuring FAM fluorescence under three sets of temperature lowering conditions with respect to the temperature range from 95°C to 35°C. Furthermore, ROX was added for use as an internal standard substance, and the value obtained by dividing the value of FAM fluorescence by the value of ROX fluorescence was used as the relative fluorescence value of FAM.

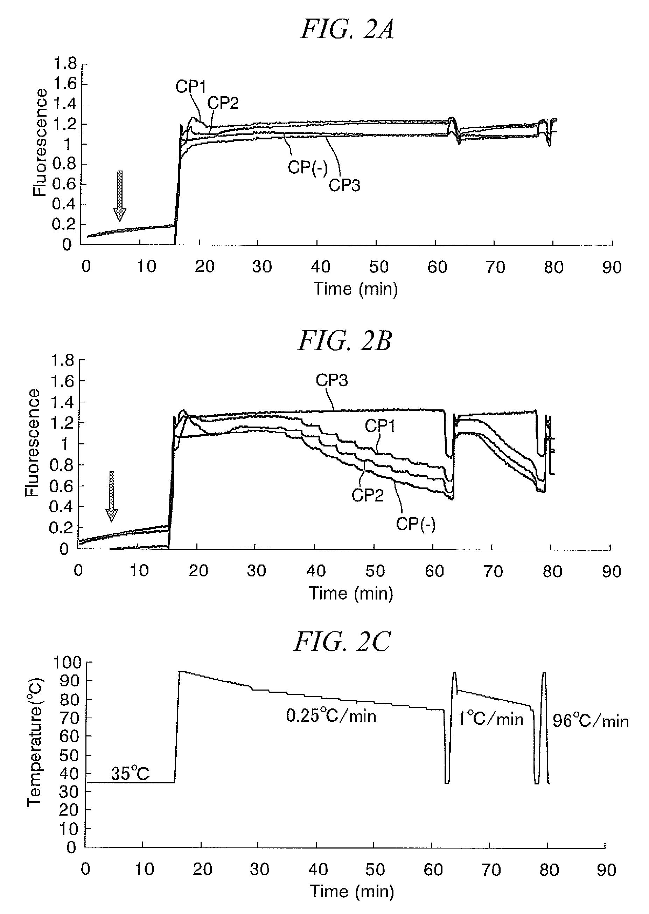

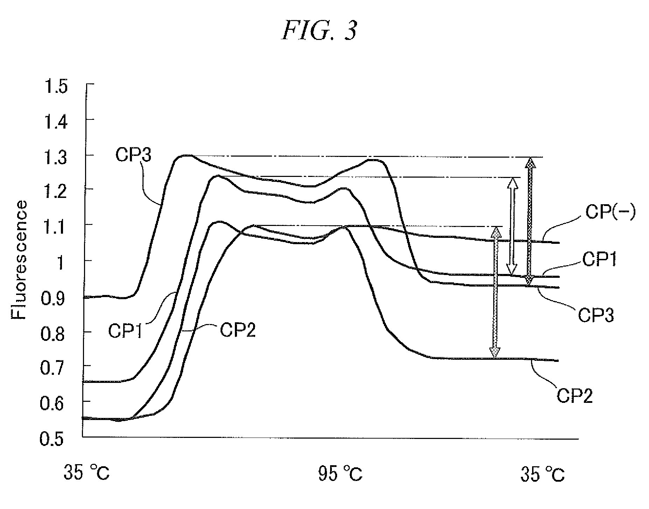

The results of real-time measurement of fluorescence intensity of the reaction solution at each temperature are shown in FIGS. 2 and 3.

In the figures, CP (-) indicates results in the case of not adding polymer, while CP1 to CP3 indicate the results of adding each polymer.

FIG. 2(A) indicates changes in fluorescence versus temperature change in the case the base sequences of the sample double-stranded nucleic acid and the reference double-stranded nucleic acid are both G12S and both are completely matching. The reference double-stranded nucleic acid competes with the sample double-stranded nucleic acid present in excess, and a strand displacement reaction occurs. In this case, FAM fluorescence is unable to be quenched by DABCYL, and FAM fluorescence remains at a constant value even if the temperature is lowered.

On the other hand, FIG. 2 (B) indicates the case of the sample double-stranded nucleic acid being of the wild type, the reference double-stranded nucleic acid being G12S, and both differing by a single base. In this case, since both the reference double-stranded nucleic acid and sample double-stranded nucleic acid each preferentially form a double strand, it is difficult for strand displacement to occur. Thus, in addition to the formation of double-stranded nucleic acid by strand recombination, FAM fluorescence is quenched by DABCYL and decreases.

FIG. 2 (C) indicates the temperature change profile. This shows that, in the case of holding for 15 minutes at 35°C and then lowering the temperature for the first time, the temperature decreased comparatively slowly at the rate of 0.25°C/minute from 95°C to 65°C. When the solution was again heated to 95°C followed by lowering the temperature a second time, the temperature decreased to 65°C at the rate of 1°C/minute. When the solution was again heated to 95°C followed by lowering the temperature for a third time, the temperature decreased to 35°C at the rate of 96°C/minute.

FIG. 3 shows an enlargement of the portion where the temperature was lowered a third time in FIG. 2 (B) (96°C/minute).

According to these results, even in the case of having added any of the polymers as in FIG. 2(A), fluorescence did not decrease and FRET was not observed at any of the temperature lowering rates. This is thought to be due to the occurrence of strand recombination between the reference double-stranded nucleic acid and excess sample double-stranded nucleic acid in all of the reactions, thereby preventing FAM fluorescence from being quenched by DABCYL.

On the other hand, in FIG. 2(B), fluorescence decreased as a result of lowering temperature when the temperature was lowered for the first time (0.25°C/minute) with the exception of the case of adding polymer CP3. This indicates that, in the case of the reactions in which polymer CP1 and polymer CP2 were added, the labeled reference double-stranded nucleic acid returned to its original state, FRET occurred and FAM fluorescence was quenched by DABCYL. During the second time the temperature was lowered as well (1°C/minute), behavior similar to that when the temperature was lowered the first time was demonstrated, and even at this temperature lowering rate, completely complementary nucleic acids preferably formed double strands in all reactions with the exception of CP3. As shown in FIG. 3, when the temperature was lowered for the third time (9C°C/minute), fluorescence decreased as a result of lowering the temperature and a single base difference was able to be identified in all reactions in which polymers were added.

Furthermore, in the case of having added polymer CP3, fluorescence did not decrease when the temperature was lowered for the first time and second time. This is thought to be due to a decrease in the Tm value of the reference double-stranded nucleic acid resulting from addition of polymer CP3, thereby preventing the formation of a double strand during the first time and second time the temperature was lowered to 65°C. The effect was observed when the temperature was lowered for a third time since a double strand was formed as a result of lowering the temperature to 35° at that time.

[Example 2]

Wild-type KRAS codon 12 (Wild) and a mutant type (G12S) were identified using the identification method of the present invention in the same manner as Example 1 using sample double-stranded nucleic acid prepared by PCR. Polymer CP2, which demonstrated the greatest effect in Example 1, was used for the cationic comb-type polymer.

The composition of the PCR reaction solution consisted of 250 nM KF primer, 250 nM KR primer, 250 $\mu$M dNTP, 1× PCR buffer and 2.5 units of Taq DNA polymerase (Takara Taq HotStart Version), and the total volume of the reaction solution was made to be 47.5 $\mu$L. 2.5 $\mu$L of template DNA having a concentration of 10 ng/$\mu$L were added to this PCR reaction solution to bring to a total reaction solution volume of 50 $\mu$L. PCR reaction conditions consisted of treating for 3 minutes at 95°C followed by carrying out 40 cycles consisting of denaturation, annealing and extension reactions comprising 95°C for 20 seconds, 57°C for 30 seconds and 72°C for 30 seconds. The base sequences of the KF primer and KR primer used are shown in Table 2. The numerals shown on the right-hand side of the table indicate the corresponding sequence numbers listed in the sequence listing. Furthermore, cancer cell-derived DNA (DLD-1 and A549) was used for the template DNA. The KRAS genotype of DLD-1 is G13D (hetero), while the KRAS genotype of A549 is the G12S mutant type (homo). These sequences were confirmed by direct sequencing.

[Table 2]

| | Base Sequence | |
|---|---|---|
| KF primer | 5'-TATAAACTTGTGGTAGTTGGAGCT | 5 |
| KR primer | 5'-TATCGTCAAGGCACTCTTGCC | 6 |

The resulting PCR reaction solution (12.4 $\mu$L), 500 nM wild-FAM (single-stranded nucleic acid labeled with FAM of the wild-type reference double-stranded nucleic acid) (1 $\mu$L), 500 nM wild-DAB (single-stranded nucleic acid labeled with DABCYL of the wild-type reference double-stranded nucleic acid) (1 $\mu$L), 2 M NaCl (1 $\mu$L), ROX (0.6 $\mu$L), 0.5 M EDTA (2 $\mu$L) and polymer CP3 (1 $\mu$L) were mixed for use as a fluorescent PHFA reaction solution. The total amount of anion was calculated from the phosphate residue concentration of nucleic acids present in the entire reaction solution, and cationic polymer was added to the reaction solution so that the amount of cationic polymer was roughly eight times the amount of anion. Two types of PCR reaction solutions were respectively prepared consisting of that in which DLD-1-derived genome (genome having both the wild-type and G13D mutant genes) was used as template (DLD-1), and that in which A549-derived genome (genome having only G12S mutant gene) was used as template. Changes in fluorescence versus temperature change of the prepared fluorescence PHFA reaction solutions were measured using a real-time PCR device in the same manner as Example 1.

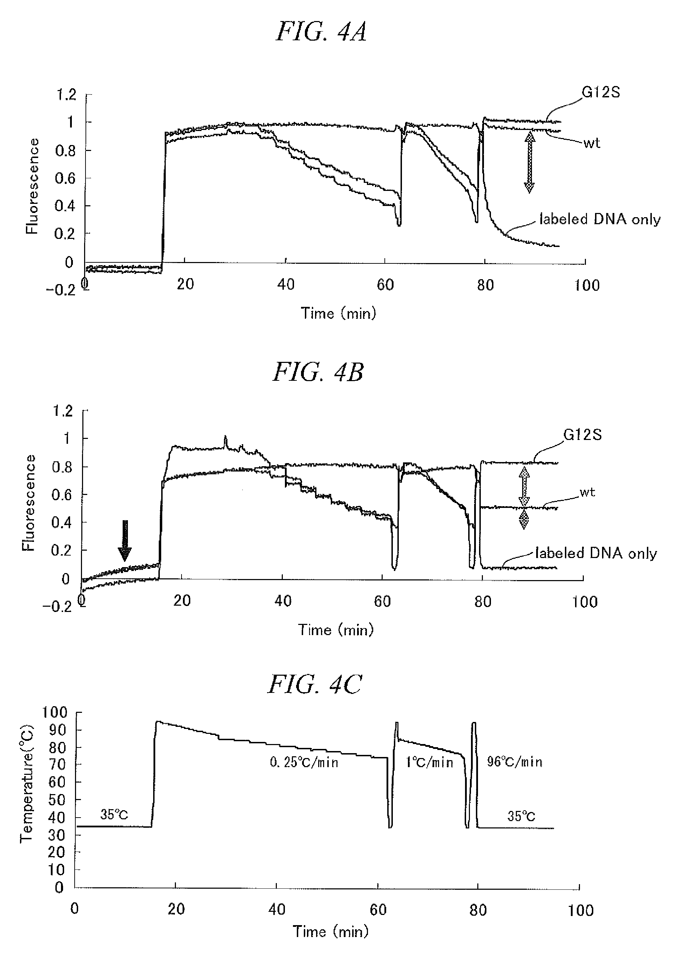

The results of real-time measurement of fluorescence intensity of the reaction solution at each temperature in the case of using wild-type labeled reference double-stranded nucleic acid are shown in FIG. 4. FIG. 4 (A) shows fluorescence behavior in the case of not adding cationic polymer, while FIG. 4 (B) shows fluorescence behavior in the case of adding cationic polymer. FIG. 4(C) indicates the temperature change profile. The experiment was conducted using three types of temperature lowering rates in the same manner as Example 1. In FIGS. 4 (A) and 4(B), "wt" indicates results for the PCR reaction solution in which the DLD-1-derived genome was used for the template, "G12S" indicates results for the PCR reaction solution in which the A549-derived genome was used for the template, and "labeled DNA only" indicates results for the solution of a PCR reaction carried out without using a genome for the template.

As a result, as shown in FIG. 4(A), in the case of using a PCR reaction solution prepared from a genome having the wild-type base sequence (DLD-1) used for the sample double-stranded nucleic acid, decreases in FAM fluorescence were not observed for any of the temperature lowering rates, and the sample double-stranded nucleic acid was able to be identified as having the same base sequence as the labeled reference double-stranded nucleic acid. On the other hand, in the case of using a PCR reaction solution prepared from a genome having the base sequence of the G12S mutant type (A549) used for the sample double-stranded nucleic acid, FAM fluorescence decreased at a low temperature in the same manner as a reaction solution to which only the labeled reference double-stranded nucleic acid was added but not the sample double-stranded nucleic acid when the temperature was lowered for the first time and second time. On the basis of this result, the sample double-stranded nucleic acid was able to be identified as not having the same base sequence as the labeled reference double-stranded nucleic acid. However, in the case of lowering the temperature for the third time (96°C/minute), FAM fluorescence did not decrease at a low temperature and differences between the two nucleic acids were unable to be identified even in the case using either of the wild type and G12S sample double-stranded nucleic acids. This is due to the formation of a heteroduplex resulting from the temperature lowering rate being rapid and preventing a distinction from being made between the wild type and G12S.

On the other hand, in FIG. 4(B) that depicts addition of cationic comb-type polymer, FAM fluorescence decreased as a result of lowering the temperature in the case of using the G12S sample double-stranded nucleic acid even when the temperature was lowered for the third time, the wild-type labeled reference double-stranded nucleic acid and the G12S sample double-stranded nucleic acid each preferentially formed a double strand, and heteroduplex formation was inhibited. On the basis of these results, even in the case of using a PCR product for the sample double-stranded nucleic acid, identifiability of single bases was observed to be maintained even when the temperature was lowered rapidly in a reaction system containing cationic comb-type polymer.

[Example 3]

An SNP said to be involved in alcohol metabolism of a gene (ALDH2) that encodes alcohol dehydrogenase was identified and detected using the identification method of the present invention. Polymer CP2, which demonstrated the greatest effect in Example 1, was used for the cationic comb-type polymer.

Reference double-stranded nucleic acids having genotypes of the A and G alleles for the SNP site were respectively prepared by ordinary chemical synthesis. The 5' end of the one of the nucleic acid strands of the reference double-stranded nucleic acid used to detect the A genotype was labeled with FAM (Glen Research Corp.) and the 3' end of the other nucleic acid strand was labeled with DABCYL (Glen Research Corp.). On the other hand, the 5' end of one of the nucleic acid strands of the reference double-stranded nucleic acid for detecting the G genotype was labeled with Alexa 594 (Glen Research Corp.) and the 3' end of the other nucleic acid strand was labeled with DABCYL (Glen Research Corp.). Each reference double-stranded nucleic acid was prepared by hybridizing the products of chemically synthesizing one strand each of the two nucleic acid strands that compose the reference double-stranded nucleic acids. The sequences of the chemically synthesized nucleic acid strands are shown in Table 3 for each genotype. In Table 3, "AL(A)" indicates the A genotype, "AL(G)" indicates the G genotype, and "Ale594" indicates the Alexa 594 label. In addition, "6-FAM", "DAB" and the numerals in the right-hand column are the same as previously defined in Table 1.

[Table 3]

| | Reference Double-Stranded Nucleic Acids | |
|---|---|---|
| AL(A) | 5'(6-FAM)-AAGAGTTGGGCGAGTACGGGCTGCAGGCATACACT A AAGTGAAAACTGTGAGTGTGGGACCTTT-3' | 7 |
| | 5'-AAAGGTCCCACACTCACAGTTTTCACTT T AGTGTATGCCTGC AGCCCGTACTCGCCCAACTCTT-(DAB)3' | 8 |
| AL (G) | 5'(6-Ale594)-AAGAGTTGGGCGAGTACGGGCTGCAGGCATACACT G AAGTGAAAACTGTGAGTGTGGGACCTTT-3' | 9 |
| | 5'-AAAGGTCCCACACTCACAGTTTTCACTT C AGTGTATGCCTGC AGCCCGTACTCGCCCAACTCTT-(DAB)3' | 10 |

On the other hand, sample double-strand nucleic acids were prepared by PCR. The composition of the PCR reaction solution consisted of 250 nM primer F, 250 nM primer R, 250 μM dNTP, 1× PCR buffer and 2.5 units of Taq DNA polymerase (Takara Taq HotStart Version), and the total volume of the reaction solution was made to be 47.5 μL. 2.5 μL of template DNA (Toyobo Co., Ltd.) having a concentration of 10 ng/μL were added to this PCR reaction solution to bring to a total reaction solution volume of 50 μL. PCR reaction conditions consisted of treating for 3 minutes at 95°C followed by carrying out 40 cycles consisting of denaturation, annealing and extension reactions comprising 95°C for 20 seconds, 57°C for 30 seconds and 72°C for 30 seconds. The base sequences of the control DNA, primer F and primer R used are shown in Table 4. The numerals shown on the right-hand side of the table indicate the corresponding sequence numbers listed in the sequence listing. Furthermore, template DNA of the A genotype or G genotype were respectively used for sample double-stranded nucleic acids having the genotype A allele homo or G allele homo. In addition, template DNA obtained by mixing equal amounts of both templates was used for sample double-stranded nucleic acid having a hetero genotype. The total amounts of DNA added were the same.

[Table 4]

| | Base Sequence | |
|---|---|---|
| Control DNA AL (A) | TCAAATTACAGGGTCAACTGCTATGATGTGTTTGGAGCCC AGTCACCCTTTGGTGGCTACAAGATGTCGGGGAGTGGCCG GGAGTGGCCGGGAGTTGGGCGAGTACGGGCTGCAGGCATA CACT A AAGTGAAAACTGTGAGTGTGGGACCTGCTGGGGGC | 11 |
| | TCAGGGCCTGTTGGGGCTTGAGGGTCTG | |
| Control DNA AL(G) | TCAAATTACAGGGTCAACTGCTATGATGTGTTTGGAGCCC AGTCACCCTTTGGTGGCTACAAGATGTCGGGGAGTGGCCG GGAGTGGCCGGGAGTTGGGCGAGTACGGGCTGCAGGCATA CACT G AAGTGAAAACTGTGAGTGTGGGACCTGCTGGGGGC TCAGGGCCTGTTGGGGCTTGAGGGTCTG | 12 |
| Primer F | 5'-AAGAGTTGGGCGAGTACGGG | 13 |
| Primer R | 5'-AAAGGTCCCACACTCACAGTTTTC | 14 |

Fluorescent PHFA reaction solutions were prepared in the same manner as Example 2 using each of the reference double-stranded nucleic acids and sample double-stranded nucleic acids prepared in this manner. Changes in fluorescence versus temperature changes of the prepared fluorescent PHFA reaction solutions were measured using the MX3000P (Stratagene). Temperature lowering conditions consisted of denaturing for 30 seconds at 95°C followed by lowering the temperature to 90°C and then lowering the temperature to 35°C at the rate of 2.5°C/second. Fluorescence intensities at 90°C and 35°C were respectively measured for FAM and Alexa594. The difference in fluorescence value between 90°C and 35°C was represented with an index value as indicated below. Here, "ΔF" is the value obtained by subtracting the fluorescence value of FAM or Alexa594 at 35°C from the fluorescence value at 90°C. In addition, the "control reaction solution" refers to a reaction solution to which sample double-stranded nucleic acid was not added (reaction solution containing only labeled reference double-stranded nucleic acid and cationic comb-type polymer).

$$Index(\%) = \Delta F[\text{reaction solution}]/\Delta F[\text{control reaction solution}] \times 100$$

Differences in the change in fluorescence for each label were standardized by dividing ΔF of the reaction solution by ΔF of the control reaction solution. Fluorescence values are temperature-dependent, and tend to become higher at low temperatures. Thus, in the case the base sequences of the sample double-stranded nucleic acid and the labeled reference double-stranded nucleic acid are the same, there are cases in which the fluorescence value at 35°C is higher than the fluorescence value at 90°C, and in this case, the index value becomes negative. In the case the index value is negative or close to zero, this indicates that strand recombination occurs between the sample double-stranded nucleic acid and the labeled reference double-stranded nucleic acid, and that the base sequences thereof are identical.

On the other hand, in the case the index value is positive and sufficiently large, this indicates that there is a difference in the base sequences of the sample double-stranded nucleic acid and the labeled reference double-stranded nucleic acid.

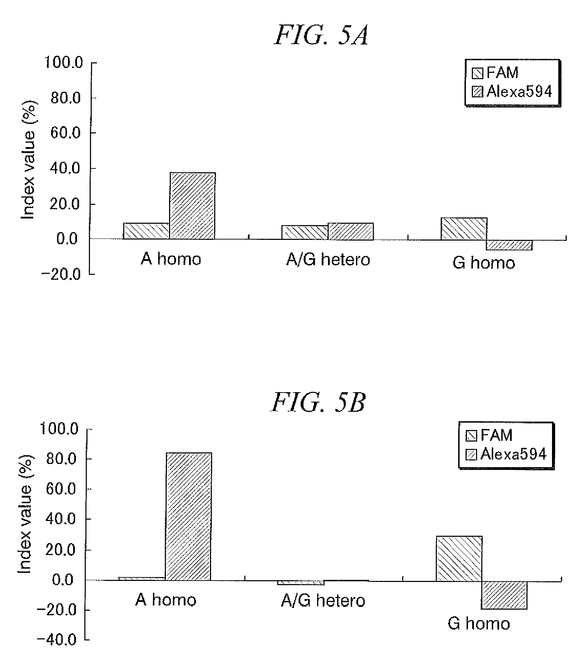

FIG. 5 shows the results of rapidly lowering the temperature (2.5°C/second) in the form of bar graphs of index values. FIG. 5 (A) shows the case of not adding the cationic comb-type polymer, while FIG. 5(B) shows the case of having added the cationic comb-type polymer. In FIG. 5, "A homo" indicates the results for using the sample double-stranded nucleic acid having the A allele homo genotype, "A/G hetero" indicates the results for using the sample double-stranded nucleic acid having the hetero genotype, and "G homo" indicates the results for using the sample double-stranded nucleic acid having the G allele homo genotype. In addition, in the drawing, the left column indicates index values for FAM (index values of a labeled double-stranded nucleic acid used to detect A allele modified with FAM), while the right column indicates index values for Alexa594 (index values of labeled double-stranded nucleic acid for detection of G allele modified with Alexa594).

As shown in FIG. 5(A), in the case of not adding cationic comb-type polymer, the index values of Alexa594 were positive and sufficiently large in the case the sample double-stranded nucleic acid was A allele homo and negative in the case of G allele homo, thereby making it possible to detect and identify the G genotype from the A genotype. However, index values of FAM were high in the same manner as in the case of A allele homo even in the case the sample double-stranded nucleic acid was G allele homo, thereby preventing detection and identification of the A genotype from the G genotype.

In contrast, as shown in FIG. 5(B), in the case of having added cationic comb-type polymer, index values of FAM were positive and sufficiently large in the case the sample double-stranded nucleic acid was G allele homo, and approached zero in the case of A allele homo, thereby making it possible to clearly identify the G genotype and the A genotype.

On the basis of these results, as a result of adding a cationic comb-type polymer to the reaction solution, even in the case of having rapidly lowered the temperature of the reaction solution, it was clearly shown that single base differences can be identified by competitive hybridization. In addition, according to the present example, it was also clearly demonstrated that, as a result of having labeled reference double-stranded nucleic acids corresponding to opposing genotypes having different fluorescent labels present simultaneously, both genotypes can be detected simultaneously.

INDUSTRIAL APPLICABILITY

Since the method for identifying a target base sequence of the present invention makes it possible to identify base sequences such as genotypes that only differ by one or several bases both rapidly (such as in just a few minutes) and with high accuracy, it can be used in fields such as clinical laboratory testing, and particularly in fields such as testing for somatic cell mutations.

## Claims

1.  A method for identifying a target base sequence, comprising:

    a thermal denaturation step for subjecting a sample double-stranded nucleic acid and a reference double-stranded nucleic acid containing the same base sequence as a target base sequence to thermal denaturation treatment in a single reaction solution,
    a temperature lowering step for carrying out competitive hybridization between the sample double-stranded nucleic acid and the reference double-stranded nucleic acid by lowering the temperature of the reaction solution after the thermal denaturation step,
    a measurement step for measuring a double-stranded nucleic acid formed by a nucleic acid strand that composed the reference double-stranded nucleic acid and a nucleic acid strand that composed the sample double-stranded nucleic acid, and
    an identification step for identifying identity between the reference double-stranded nucleic acid and the sample double-stranded nucleic acid based on measurement results obtained from the measurement step; wherein,

the temperature lowering step is carried out in the presence of a cationic comb polymer
and a temperature lowering rate of the reaction solution in the temperature lowering step is 0.2°C/second to 3°C/second.

2. The method for identifying a target base sequence according to claim 1, wherein the cationic comb polymer has a main chain that is a polymer chain containing a cationic group and a side chain that is a hydrophilic group.

3. The method for identifying a target base sequence according to claim 2, wherein the main chain of the cationic comb polymer is polylysine.

4. The method for identifying a target base sequence according to claim 2, wherein the side chain of the cationic comb polymer is dextran.

5. The method for identifying a target base sequence according to claim 2, wherein the main chain of the cationic comb polymer contains a guanidyl group.

6. The method for identifying a target base sequence according to claim 2, wherein the molecular weight of the main chain moiety of the cationic comb polymer is 5,000 or more.

7. The method for identifying a target base sequence according to claim 1, wherein the strand lengths of the sample double-stranded nucleic acid and the reference double-stranded nucleic acid are equal.

8. The method for identifying a target base sequence according to claim 1, wherein the target base sequence is a base sequence that is homologous with a region containing a mutation site of a specific genotype of a gene mutation.

9. The method for identifying a target base sequence according to claim 1, wherein the two nucleic acid strands that compose the reference double-stranded nucleic acid are respectively labeled with mutually different types of labeling substances.

10. The method for identifying a target base sequence according to claim 9, wherein among the two nucleic acid strands that compose the reference double-stranded nucleic acid, energy transfer is possible between a labeling substance used to label one of the nucleic acid strands and a labeling substance used to label the other nucleic acid strand.

11. The method for identifying a target base sequence according to claim 9, wherein the two nucleic acid strands that compose the reference double-stranded nucleic acid are both labeled with a fluorescent substance.

12. The method for identifying a target base sequence according to claim 9, wherein among the two nucleic acid strands that compose the reference double-stranded nucleic acid, one of the nucleic acid strands is labeled with a fluorescent substance, while the other nucleic acid strand is labeled with a quenching substance.

13. The method for identifying a target base sequence according to claim 9, wherein among the two nucleic acid strands that compose the reference double-stranded nucleic acid, one of the nucleic acid strands is labeled with a labeling substance able to bind to a solid phase carrier.

**Patentansprüche**

1. Verfahren zum Identifizieren einer Zielbasissequenz, umfassend:

einen thermischen Denaturierungsschritt zum Unterwerfen einer doppelsträngigen Probenukleinsäure und einer doppelsträngigen Bezugsnukleinsäure, die dieselbe Basissequenz wie eine Zielbasissequenz enthält, einer thermischen Denaturierungsbehandlung in einer einzigen Reaktionslösung,
einen Temperaturreduktionsschritt zum Ausführen einer Konkurrenzhybridisierung zwischen der doppelsträngigen Probenukleinsäure und der doppelsträngigen Bezugsnukleinsäure durch Reduzieren der Temperatur der Reaktionslösung nach dem thermischen Denaturierungsschritt,
einen Messschritt zum Messen einer doppelsträngigen Nukleinsäure, die durch einen Nukleinsäurestrang, der die doppelsträngige Bezugsnukleinsäure bildet, und einen Nukleinsäurestrang, der die doppelsträngige Probenukleinsäure bildet, gebildet wird, und

einen Identifizierungsschritt zum Identifizieren der Identität zwischen der doppelsträngigen Bezugsnukleinsäure und der doppelsträngigen Probenukleinsäure auf der Basis von Messbestimmungsergebnissen, die aus dem Messschritt erhalten werden; wobei

der Temperaturreduktionsschritt in Gegenwart eines kationischen Kammpolymers durchgeführt wird und eine Temperaturreduktionsrate der Reaktionslösung im Temperaturreduktionsschritt 0,2 °C/Sekunde bis 3 °C/Sekunde beträgt.

2. Verfahren zum Identifizieren einer Zielbasissequenz nach Anspruch 1, wobei das kationische Kammpolymer eine Hauptkette, die eine Polymerkette ist, die eine kationischen Gruppe enthält, und eine Seitenkette aufweist, die eine hydrophile Gruppe ist.

3. Verfahren zum Identifizieren einer Zielbasissequenz nach Anspruch 2, wobei die Hauptkette des kationischen Kammpolymers Polylysin ist.

4. Verfahren zum Identifizieren einer Zielbasissequenz nach Anspruch 2, wobei die Seitenkette des kationischen Kammpolymers Dextran ist.

5. Verfahren zum Identifizieren einer Zielbasissequenz nach Anspruch 2, wobei die Hauptkette des kationischen Kammpolymers eine Guanidylgruppe enthält.

6. Verfahren zum Identifizieren einer Zielbasissequenz nach Anspruch 2, wobei das Molekulargewicht des Hauptkettenanteils des kationischen Kammpolymers 5 000 oder mehr beträgt.

7. Verfahren zum Identifizieren einer Zielbasissequenz nach Anspruch 1, wobei die Stranglängen der doppelsträngigen Probenukleinsäure und der doppelsträngigen Bezugsnukleinsäure gleich sind.

8. Verfahren zum Identifizieren einer Zielbasissequenz nach Anspruch 1, wobei die Zielbasissequenz eine Basissequenz ist, die mit einer Region homolog ist, die einen Mutationsort eines spezifischen Genotyps einer Genmutation enthält.

9. Verfahren zum Identifizieren einer Zielbasissequenz nach Anspruch 1, wobei die beiden Nukleinsäurestränge, die die doppelsträngige Bezugsnukleinsäure bilden, jeweils mit gegenseitig verschiedenen Typen von Markiersubstanzen markiert sind.

10. Verfahren zum Identifizieren einer Zielbasissequenz nach Anspruch 9, wobei unter den beiden Nukleinsäuresträngen, die die doppelsträngige Bezugsnukleinsäure bilden, eine Energieübertragung zwischen einer Markiersubstanz, die zum Markieren eines der Nukleinsäurestränge verwendet wird, und einer Markierungssubstanz, die zum Markieren des anderen Nukleinsäurestrang verwendet wird, möglich ist.

11. Verfahren zum Identifizieren einer Zielbasissequenz nach Anspruch 9, wobei die beiden Nukleinsäurestränge, die die doppelsträngige Bezugsnukleinsäure bilden, beide mit einer fluoreszierenden Substanz markiert sind.

12. Verfahren zum Identifizieren einer Zielbasissequenz nach Anspruch 9, wobei unter den beiden Nukleinsäuresträngen, die die doppelsträngige Bezugsnukleinsäure bilden, einer der Nukleinsäurestränge mit einer fluoreszierenden Substanz markiert ist, während der andere Nukleinsäurestrang mit einer Quenchsubstanz markiert ist.

13. Verfahren zum Identifizieren einer Zielbasissequenz nach Anspruch 9, wobei unter den beiden Nukleinsäuresträngen, die die doppelsträngige Bezugsnukleinsäure bilden, einer der Nukleinsäurestränge mit einer Markiersubstanz markiert ist, die in der Lage ist, sich an einen Festphasenträger zu binden.

**Revendications**

1. Procédé d'identification d'une séquence de base cible, comprenant:

une étape de dénaturation thermique pour soumettre un acide nucléique double brin échantillon et un acide nucléique double brin de référence contenant la même séquence de base que la séquence de base cible à un traitement de dénaturation thermique dans une solution réactionnelle unique,

une étape de réduction de température pour conduire une hybridation compétitive entre l'acide nucléique double brin échantillon et l'acide nucléique double brin de référence en réduisant la température de la solution réactionnelle après l'étape de dénaturation thermique,

une étape de mesure pour mesurer un acide nucléique double brin formé par un brin d'acide nucléique qui composait l'acide nucléique double brin de référence et un brin d'acide nucléique qui composait l'acide nucléique double brin échantillon, et

une étape d'identification pour l'identification de l'identité entre l'acide nucléique double brin de référence et l'acide nucléique double brin échantillon sur la base des résultats de mesure obtenus à partir de l'étape de mesure; où,

l'étape de réduction de température est conduite en présence d'un polymère cationique à structure en peigne et une étape de réduction de température de la solution réactionnelle dans l'étape de réduction de température est de 0,2°C/seconde à 3°C/seconde.

2. Procédé d'identification d'une séquence de base cible selon la revendication 1, où le polymère cationique à structure en peigne présente une chaîne principale qui est une chaîne polymère contenant un groupe cationique et une chaîne latérale qui est un groupe hydrophile.

3. Procédé d'identification d'une séquence de base cible selon la revendication 2, où la chaîne principale du polymère cationique à structure en peigne est la polylysine.

4. Procédé d'identification d'une séquence de base cible selon la revendication 2, où la chaîne latérale du polymère cationique à structure en peigne est le dextrane.

5. Procédé d'identification d'une séquence de base cible selon la revendication 2, où la chaîne principale du polymère cationique à structure en peigne contient un groupe guanidyle.

6. Procédé d'identification d'une séquence de base cible selon la revendication 2, où le poids moléculaire de la fraction chaîne principale du polymère cationique à structure en peigne est 5 000 ou plus.

7. Procédé d'identification d'une séquence de base cible selon la revendication 1, où les longueurs de brin de l'acide nucléique double brin échantillon et de l'acide nucléique double brin de référence sont égales.

8. Procédé d'identification d'une séquence de base cible selon la revendication 1, où la séquence de base cible est une séquence de base qui est homologue à une région contenant un site de mutation d'un génotype spécifique d'une mutation de gène.

9. Procédé d'identification d'une séquence de base cible selon la revendication 1, où les deux brins d'acide nucléique qui composent l'acide nucléique double brin de référence sont respectivement marqués avec des types mutuellement différents de substances de marquage.

10. Procédé d'identification d'une séquence de base cible selon la revendication 9, où parmi les deux brins d'acide nucléique qui composent l'acide nucléique double brin de référence, le transfert d'énergie est possible entre une substance de marquage utilisée pour marquer l'un des brins d'acide nucléique et une substance de marquage utilisée pour marquer l'autre brin d'acide nucléique.

11. Procédé d'identification d'une séquence de base cible selon la revendication 9, où les deux brins d'acide nucléique qui composent l'acide nucléique double brin de référence sont tous deux marqués avec une substance fluorescente.

12. Procédé d'identification d'une séquence de base cible selon la revendication 9, où parmi les deux brins d'acide nucléique qui composent l'acide nucléique double brin de référence, l'un des brins d'acide nucléique est marqué avec une substance fluorescente, tandis que l'autre brin d'acide nucléique est marqué avec une substance de désactivation.

13. Procédé d'identification d'une séquence de base cible selon la revendication 9, où parmi les deux brins d'acide nucléique qui composent l'acide nucléique double brin de référence, l'un des brins d'acide nucléique est marqué avec une substance de marquage capable de se lier à un support à phase solide.

FIG. 1A — Thermal denaturation → Gradual temperature lowering

FIG. 1B — Thermal denaturation → Rapid temperature lowering

FIG. 1C — Thermal denaturation → Addition of cationic comb-type polymer, rapid temperature lowering

Sample double-stranded nucleic acid

Reference double-stranded nucleic acid

## FIG. 2A

## FIG. 2B

## FIG. 2C

*FIG. 3*

## FIG. 4A

## FIG. 4B

## FIG. 4C

## FIG. 5A

## FIG. 5B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2982304 B
- JP 2003174882 A
- JP 2001078769 A
- JP 4178194 B
- JP 2008278779 A
- JP S5993099 A
- JP S59148798 A
- JP S59204200 A
- JP 2010075297 A

### Non-patent literature cited in the description

- **CHEN et al.** *Analytical Biochemistry,* 1991, vol. 195, 51-56
- **JACOBSON et al.** *Oncogene,* 1994, vol. 9, 553-563
- **NEWTON et al.** *Nucleic Acids Research,* 1989, vol. 17, 2503-2516
- **WU et al.** *Proceedings of the National Academy of Sciences of the United States of America,* 1989, vol. 86, 2757-2760
- **NOLLAU et al.** *Clinical Chemistry,* 1997, vol. 43, 1114-1128
- **OGINO et al.** *The Journal of Molecular Diagnostics,* 2005, vol. 7, 413-421
- **KRYPUY et al.** *BMC Cancer,* 2006, vol. 6, 295
- **WALLACE et al.** *Proceedings of the National Academy of Sciences of the United States of America,* 1983, vol. 80, 278-282
- **TADA et al.** *Clinica Chimica Acta,* 2002, vol. 324, 105
- **OKA et al.** *Nucleic Acids Research,* 1994, vol. 22 (9), 1541-1547
- **MANAK.** DNA Probes. Stockton Press, 1993, 255-291
- *PCR Methods and Applications,* 1995, vol. 4, 357-362
- *Nature Biotechnology,* 1998, vol. 16, 49-53
- Nonisotopic DNA Probe Techniques. Academic Press, 1992
- *Nucleic Acids Research,* 2002, vol. 30 (9), 2089-2195
- *Biotechniques,* 1998, vol. 24, 484-489
- *Science,* 1987, vol. 238, 336-3341
- *PCR Methods and Applications,* 1992, vol. 2, 34-40